(19) Europäisches Patentamt
European Patent Office
Office européen des brevets

(11) Publication number: **0 307 827 A2**

(12) # EUROPEAN PATENT APPLICATION

(21) Application number: 88114828.2

(22) Date of filing: 09.09.88

(51) Int. Cl.[4]: C08B 37/02 , C08B 31/16 , C07F 17/00 , A61K 31/715 , A61K 31/295

(30) Priority: 15.09.87 JP 230479/87
20.11.87 JP 294824/87

(43) Date of publication of application:
22.03.89 Bulletin 89/12

(84) Designated Contracting States:
AT BE CH DE FR GB IT LI LU NL SE

(71) Applicant: KURARAY CO., LTD.
1621 Sakazu
Kurashiki-City Okayama Prefecture 710(JP)

(72) Inventor: Miki, Shuji
2047-1, Sakazu
Kurashiki-City(JP)
Inventor: Mori, Fumio
332-3, Ojima
Kurashiki-City(JP)
Inventor: Konishi, Michiko
11-23, Kaminakano 2-chome
Okayama-City(JP)
Inventor: Okada, Masafumi
1562, Sakazu
Kurashiki-City(JP)
Inventor: Nishida, Takashi
3-9, Kurashikihaitsu
Kurashiki-City(JP)
Inventor: Tashiro, Tazuko
6-2, Matsubara 2-chome Setagaya-ku
Tokyo-City(JP)
Inventor: Tsukagoshi, Shigeru
13-17, Shimoshakujii 6-chome
Nerima-ku Tokyo-City(JP)

(74) Representative: Patentanwälte Deufel- Schön- Hertel- Lewald- Otto
Isartorplatz 6
D-8000 München 2(DE)

(54) **Novel macromolecular complexes, process for producing same and medicinal use of such complexes.**

(57) A dextran or hydroxyethyl starch derivative having one or more carboxylated active sites resulting from reaction with an aliphatic di-, tri- or tetrabasic acid or a reactive derivative thereof, wherein one hydrogen atom of the group of the formula

$$-\overset{\displaystyle O}{\overset{\|}{C}}-OH$$

occurring at said active sites is partly substituted by a group of the general formula

EP 0 307 827 A2

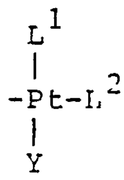

or

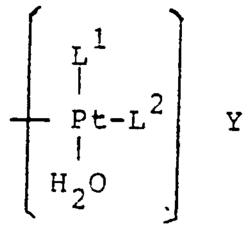

wherein $L^1$ and $L^2$ each independently is ammine or a unidentate ligand amine or combinedly represent a bidentate ligand amine and Y is an anionic ligand, and/or two hydrogen atoms of two $-\overset{O}{\overset{\|}{C}}-OH$ groups bound to one and the same carbon atom or to two neighboring carbon atoms as occurring at said active sites are, each independently, partly substituted by a group of the general formula

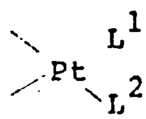

wherein $L^1$ and $L^2$ are as defined above, and a pharmacologically acceptable salt thereof.

The above compound has anticancer activity and is useful as anticancer agents.

# Novel macromolecular complexes, process for producing same and medicinal use of such complexes

## Background of the Invention

This invention relates to novel macromolecular complexes and, more particularly, to water-soluble macromolecular complexes derived from cis-platinum(II) complexes.

This invention also relates to a process for producing the novel macromolecular complexes, and to a midicinal use of said novel macromolecular complexes.

Since B. Rosenberg et al. discovered in 1969 the fact that cis-dichlorodiammineplatinum(II), a platinum complex called cisplatin by generic name, has anticancer activity against Sarcoma 180, intensive research and development works have hitherto been conducted on cancer chemotherapy using platinum complexes in various countries and now platinum complexes as unique broad-spectrum anticancer agents are attracting much attention. Reportedly, however, cisplatin has very strong nephrotoxicity and, due to its accumulability, causes gastrointestinal disorders, hypofunction of the bone- marrow, hearing impairment and so forth and, furthermore, causes severe nausea and vomiting, among others, at the time of administration, causing unimaginably severe pain in patients. In addition, the solubility of cisplatin in water is as low as about 1 mg/ml and therefore it is difficult to formulate therapeutically effective dosage forms containing it. In spite of these drawbacks, cisplatin is currently in use as an essential means in the treatment of testicular tumor, ovarian cancer, bladder cancer, head and neck tumors, etc., but its use is much restricted as a matter of course. On the other hand, those cis-dichloroplatinum(II) complexes that are known to have anticancer activity, such as cis-dichloro(1,2-diaminocyclohexane)platinum(II), cis-dichloro(2-aminomethylpyridine)-platinum(II) and cis-dichlorobis(cyclopentylamine)platinum(II), are still less water-soluble than cisplatin. Accordingly, a number of investigations have been conducted in various countries to find out platinum complex anticancer agents free from such drawbacks of cis-dichloroplatinum(II) complexes and, as a result, there have been developed, low molecular platinum complexes for example, 1,1-cyclobutanedicarboxylatodiammineplatinum(II) (carboplatin by generic name), cis-dichloro-trans-dihydroxobis(isopropylamine)platinum- (IV), R-1,1-cyclobutanedicarboxylato(2-aminomethylpyrrolidine)-platinum(II), (glycolato-O,O′)-diammineplatinum(II), 1,1-cyclobutanedicarboxylato(2-methyl-1,4-butanediamine)platinum(II) and oxalato(1R,2R-cyclohexanediamine)platinum(II).

It has also been reported that compositions containing coupling products between organic macromolecules having a molecular weight of 5,000-60,000, such as polyamino acids and anionic polysaccharides, and platinum or palladium complexes are suited for use in the treatment of tumors and for the treatment and prevention of trypanosomiasis (cf. Japanese Kokai Tokkyo Koho No. 59-116221, USP 4584392, USP 4673754). Also known are complexes of carboxymethyldextran or poly-L-glutamic acid with cisplatin or cis-diammineaquaplatinum (II) nitrate and complexes of trimellitic acid-esterified polymers with platinum complexes [Cancer Biochem. Biophys., **8**, 277-287, 289-298 (1986); and UK Patent Application GB 2168063A].

The low-molecular platinum complexes mentioned above can hardly be said to be satisfactory with respect to reduction in side-effects, indications and water-solubility, among others. For the reasons mentioned above, it is difficult to expect that some novel anticancer agent free from various drawbacks as mentioned above would be born from among low-molecular platinum complexes.

The above-mentioned macromolecular complexes can hardly be said to be satisfactory in terms of anticancer activity, toxicity, solubility in water and/or uniformity of structure.

## Summary of the Invention

It is an object of the invention to provide a novel water-soluble compound which shows side-effects to a less extent, is broader in anticancer spectrum and has better anticancer activity as compared with the conventional platinum complex anticancer agents.

Another object of the invention is to provide a process for producing said novel compound.

A further object of the invention is to provide a pharmaceutical composition containing said novel compound as well as a method of treating cancer which comprises administering said novel compound.

In accordance with the present invention, the above objects are accomplished by providing:

(1) a dextran or hydroxyethyl starch derivative having one or more carboxylated active sites resulting

from reaction with an aliphatic di-, tri- or tetrabasic acid or a reactive derivative thereof, wherein one hydrogen atom of the group of the formula

$$-\overset{\overset{\displaystyle O}{\|}}{C}-OH$$

occurring at said active sites is partly substituted by a group of the general formula

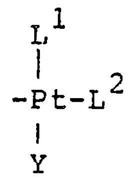 or 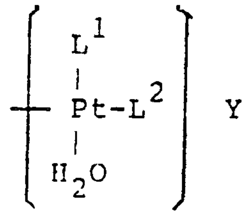

wherein $L^1$ and $L^2$ each independently is ammine or a unidentate ligand amine or combinedly represent a bidentate ligand amine and Y is an anionic ligand, and/or two hydrogen atoms of two $-\overset{\overset{\displaystyle O}{\|}}{C}-OH$ groups bound to one and the same carbon atom or to two neighboring carbon atoms as occurring at said active sites are, each independently, partly, substituted by a group of the general formula

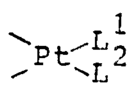

wherein $L^1$ and $L^2$ are as defined above, and/or a pharmacologically acceptable salt thereof (hereinafter these compounds are referred to as "macromolecular complexes");

(2) a process for producing a dextran or hydroxyethyl starch derivative having one or more carboxylated active sites resulting from reaction with an aliphatic di-, tri- or tetrabasic acid or a reactive derivative thereof, wherein one hydrogen atom of the group of the formula

$$-\overset{\overset{\displaystyle O}{\|}}{C}-OH$$

occurring at said active sites is partly substituted by a group of the general formula

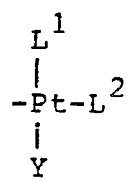 or 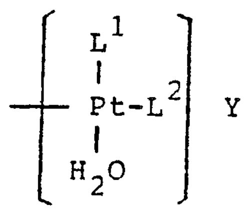

wherein $L^1$, $L^2$ and Y are as defined above, and/or two hydrogen atoms of two $-\overset{\overset{\displaystyle O}{\|}}{C}-OH$ groups bound to one and the same carbon atom or to two neighboring carbon atoms as occurring at said active sites are, each independently, partly substituted by a group of the general formula

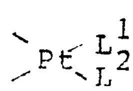

wherein $L^1$ and $L^2$ are as defined above, and/or a salt thereof which comprises reacting dextran or hydroxyethyl starch with an aliphatic di-, tri- or tetrabasic acid or a reactive derivative thereof, then reacting the thus-obtained activated dextran or hydroxy ethyl starch with a cis-platinum(II) complex of the general formula

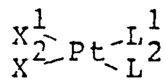

wherein $L^1$ and $L^2$ are as defined above and $X^1$ and $X^2$ each is nitrato or hydroxo or combinedly represent sulfato, to obtain a dextran or hydroxyethyl starch derivative having one or more carboxylated active sites resulting from reaction with an aliphatic di-, tri- or tetrabasic acid or a reactive derivative thereof, wherein one hydrogen atom of the group of the formula

$$-\overset{O}{\overset{\|}{C}}-OH$$

occurring at said active sites is partly substituted by a group of the general formula

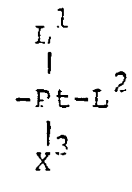

or

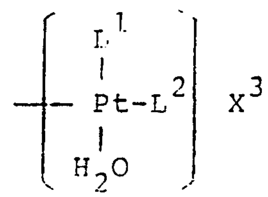

wherein $L^1$ and $L^2$ are as defined above and $X^3$ is nitrato or hydroxo or sulfato (said sulfato being the remainder sulfato resulting from binding of one of $X^1$ and $X^2$ to the activated dextran or hydroxyethyl starch), and/or two hydrogen atoms of two $-\overset{O}{\overset{\|}{C}}-OH$ groups bound to one and the same carbon atom or to two neighboring carbon atoms as occurring at said active sites are, each independently, partly substituted by a group of the general formula

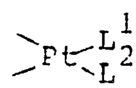

wherein $L^1$ and $L^2$ are as defined above, and/or a salt thereof, and, as required, reacting the thus-obtained dextran or hydroxyethyl starch derivative and/or a salt thereof with an anion-forming compound, to obtain a dextran or hydroxyethyl starch derivative having one or more carboxylated active sites resulting from reaction with an aliphatic di-, tri- or tetrabasic acid or a reactive derivative thereof, wherein one hydrogen atom of the group of the formula

$$-\overset{O}{\overset{\|}{C}}-OH$$

occurring at said active sites is partly substituted by a group of the general formula

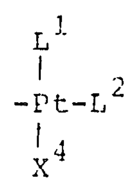

or

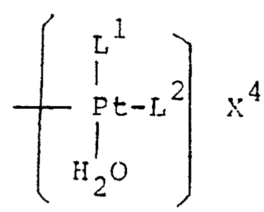

wherein $L^1$ and $L^2$ are as defined above and $X^4$ is an anionic ligand, and/or two hydrogen atoms of

two $-\overset{O}{\overset{\|}{C}}-OH$ groups bound to one and the same carbon atom or to two neighboring carbon atoms as occurring at said active sites are, each independently, partly substituted by a group of the general formula

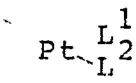

wherein L' and $L^2$ are as defined above, and/or a salt thereof;

(3) a pharmaceutical composition comprising a therapeutically effective amount of at least one of the macromolecular complexes and a pharmaceutically acceptable carrier therefor, and

(4) a method of treating cancer which comprises administering a therapeutically effective amount of at least one of the macromolecular complexes.

## Detailed Description of the Preferred Embodiments

### Drawings

Figs. 1-7 show infrared absorption spectra (FT-IR) of the macromolecular complexes obtained in Examples 1-7 which are to be rentioned later herein, respectively,. Figs. 8-12 show infrared absorption spectra (FT-IR) of the macromolecular complexes obtained in Examples 9-13, respectively. In these figures, the abscissa indicates the wave number ($cm^{-1}$) and ordinate indicates the transmittance (%).

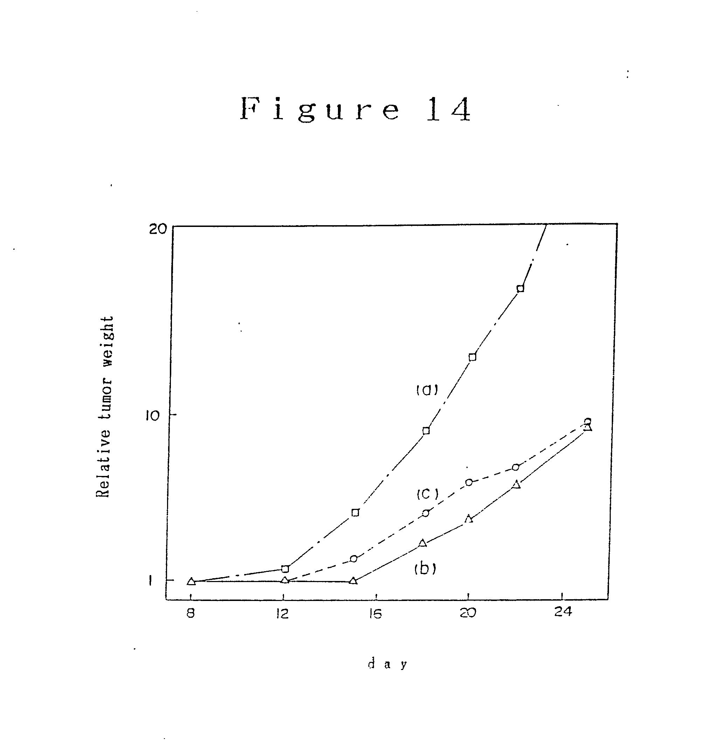

Figs. 13 and 14 show the tumor growth inhibiting effects of certain test substances on mouse B16 melanoma as found in Test Example 2. In these figures, the abscissa indicates the time in days after transplantation of cancer cells and the ordinate indicates the relative tumor weight (the ratio of an estimated tumor weight up to day 24 to the estimated tumor weight on day 8).

The lines (a), (b) and (c) in Fig. 13 represent changes in relative tumor weight after transplantation in the control group, Example 2 complex 180 mg/kg/dose group and cisplatin 3 mg/kg/dose group, respectively. The lines (a), (b) and (c) in Fig. 14 represent changes in relative tumor weight after transplantation in the control group, Example 12 complex 125 mg/kg/dose group and cisplatin 3 mg/kg/dose group.

### Macromolecular Complexes

L' and $L^2$ appearing in the above formulas are now described in further detail. As the unidentate amine represented by each of L' and $L^2$, there may be mentioned n-propylamine, n-butylamine, n-hexylamine, n-octylamine, isopropylamine, isopentylamine, oxo-2-aminonorbornane, cyclopropylamine, cyclobutylamine, cyclopentylamine, cyclohexylamine, cycloheptylamine, aniline, alanine and glycine, among others. As the bidentate amine formed combinedly by L' and $L^2$, there may be mentioned 1,2-diaminocyclopentane, 1,2-diaminocyclohexane, 1,2-diaminocycloheptane, 1,2-diaminocyclooctane, 1-amino-1-aminomethylcyclohexane, 1-amino-1-aminomethylcyclopentane, 1-amino-1-aminomethylcyclooctane, 1-amino-2-aminomethylcyclohexane, 1-amino-2-aminomethyl-3,3,5-trimethylcyclopentane, 1,1-bis(aminomethyl)cyclohexane, 1,2-diaminoadamantane, exo-cis-2,3-diaminobicyclo[2.2.1]heptane, o-phenylenediamine, ethylenediamine, 1,2-dicarboxyethylenediamine, 1,2-bis(4-hydroxyphenyl)ethylenediamine, 1,2-bis(3,4-dimethoxyphenyl)-ethylenediamine, 1,2-bis(4-methoxyphenyl)ethylenediamine, N,N'-dimethylethylenediamine, N,N'-diethylethylenediamine, N-ethylethylenediamine, 1,2-diaminopropane, 1,2-diamino-2-methylpropane, 1,2-diamino-2-ethylpropane, 1,2-diamino-2-isobutylpropane, 1,2-diamino-2-methylbutane, 1,2-diamino-2-ethylbutane, 1,3-diaminobutane, 1,3-diaminopentane, 2,4-diaminopentane, 1,3-diamino-1,3-diphenylpropane, 1,3-diamino-2-methyl-2-ethylpropane, 2-methyl-1,4-butanediamine, 1,3-diamino-2,2-dibenzylpropane, s-stilbenediamine, 2-aminomethylpyridine, 2-(1-aminoethyl)pyridine, 2-(methylaminomethyl)pyridine, 2-aminomethylpiperidine, N-(2-aminoethyl)piperidine, 2-aminomethylpyrrolidine, 2-(1-aminoethyl)pyrrolidine, N-ethyl-2-aminomethylpyrrolidine, N-(2-aminoethyl)pyrrolidine, and the like. These bidentate amines include geometrical or optical isomers thereof when such isomers exist. As the anionic ligand represented by each of Y and $X^4$, there may be mentioned, for example, hydroxo, nitrato, nitro, sulfato, ethylsulfato, sulfito, chloro, bromo, iodo, carbonato, hydrogen-carbonato, phosphato, methylphosphonato, borato, tetraborato, n-butylboronato, sulfamato, bromato, chromato, manganato, methanesulfonato, acetato, monobromoacetato,

monochloroacetato, trichloroacetato, acetoacetato, pyruvato, lactato, glucuronato, gluconato, oxalato, malonato, succinato and benzoato..

As the pharmacologically acceptable salts, which are included among the macromolecular complexes according to the invention, there may be mentioned, for example, salts with alkali metals such as sodium and potassium; salts with alkaline earth metals such as calcium and barium; and salts with tertiary amines such as pyridine, triethylamine and tri-n-butylamine.

The macromolecular complexes according to the invention can be produced in accordance with the above-mentioned process, which may be represented schematically in terms of reaction formulas as follows (dextran being represented by Dex-OH, and the activated dextran obtained by partial introduction of a carboxylic acid onto hydroxyl groups of dextran using an aliphatic di- to tetrabasic acid or a reactive derivative thereof being represented by Dex-O-Ⓐ): (1) When the activated dextran as obtained by partial introduction of a carboxylic acid into dextran, an active site of which has the group $-\overset{O}{\overset{\|}{C}}-OH$, is used:

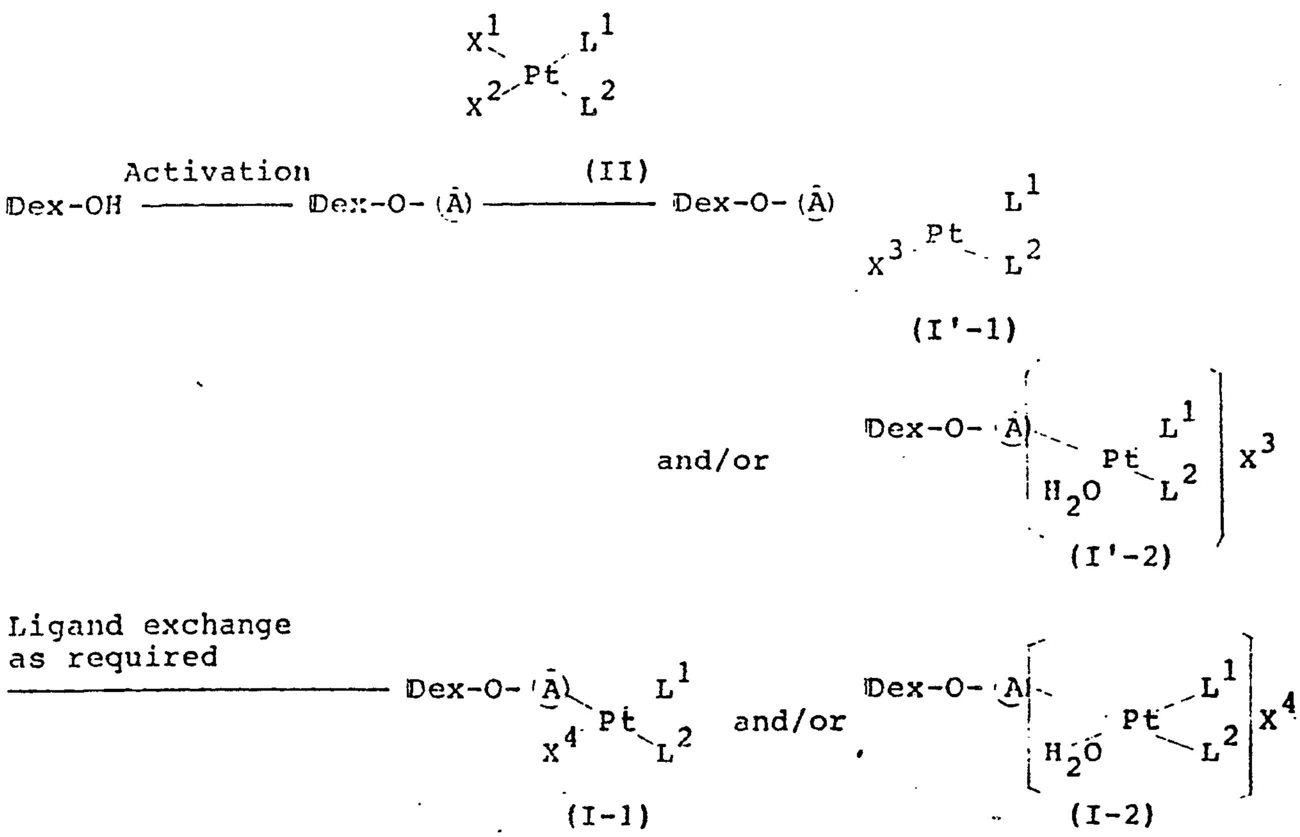

(II)
(I'-1)
and/or
(I'-2)
and/or
(I-1)
(I-2)

(2) When the activated dextran as obtained by partial introduction of a carboxylic acid into dextran, an active site of which has two more $-\overset{O}{\overset{\|}{C}}-OH$ groups, is used:

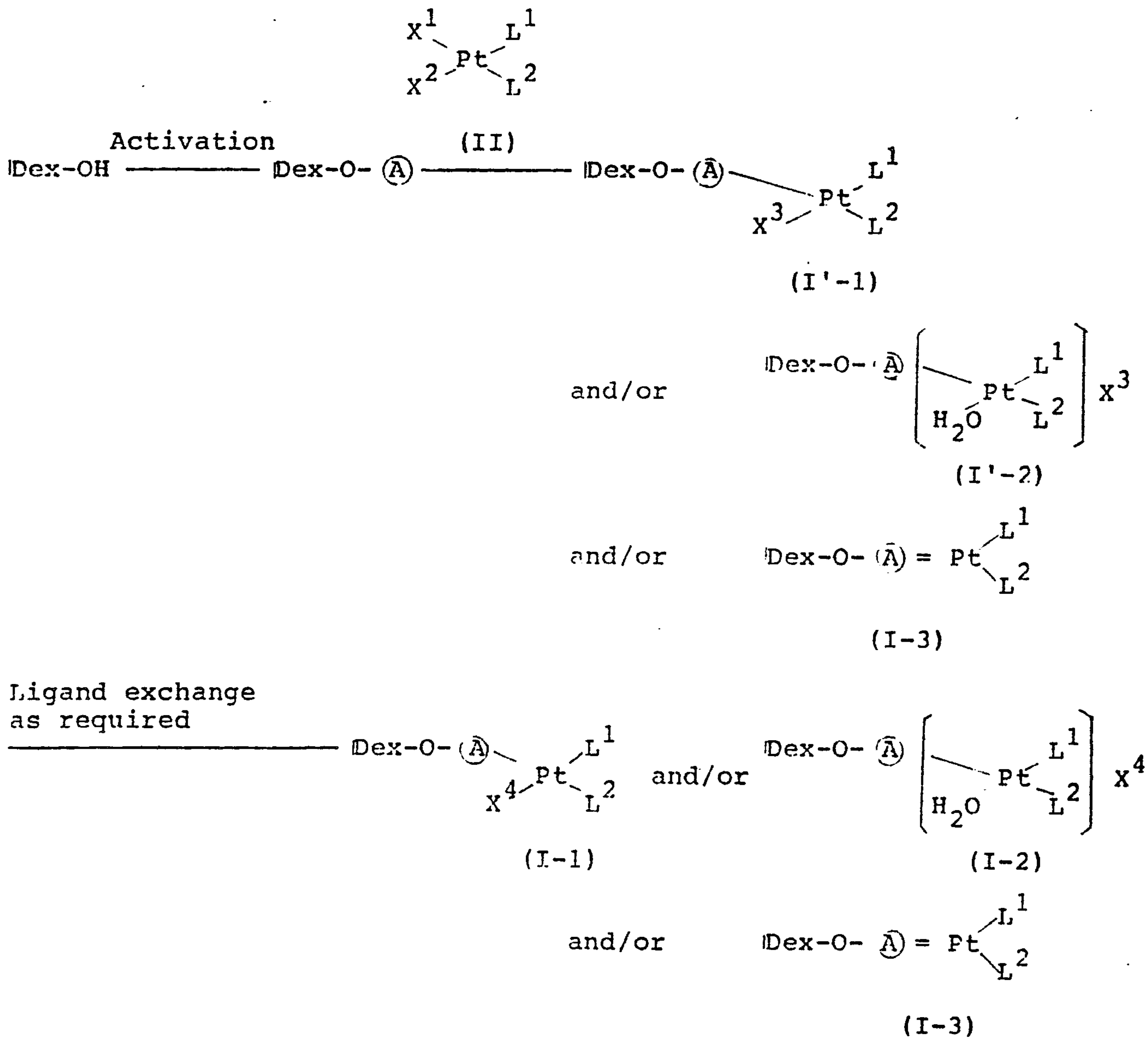

(II)
(I'-1)
and/or
(I'-2)
and/or
(I-3)
(I-1)
and/or
(I-2)
and/or
(I-3)

In the above, formulas, $L^1$, $L^2$ $X^1$, $X^2$, $X^3$ and $X^4$ are as defined above. The general formulas (I'-1), (I'-2) and (I-3) represent the macromolecular complexes according to the invention as obtained as a result of partial reaction of the respective activated dextran species with a cis-platinum(II) complex of general formula (II), while the general formulas (I-1) and (I-2) represent the macromolecular complexes according to the invention as obtained by subjecting the macromolecular complex of general formula (I'-1) and the macromolecular complex of general formula (I'-2), respectively, to ligand exchange as required. The general formulas (I-2) and (I'-2) represent those macromolecular complexes that are supposedly formed in the respective reactions or in the step of separation and purification to be mentioned later herein in which water is used. The sites of Pt binding to activated pullulan are represented by

$$-\overset{O}{\overset{\|}{C}}-O-Pt.$$

The reaction formulas given for the synthesis of dextran complexes are also applicable to the production of complexes starting with hydroxyethyl starch except that Dex-OH and Dex-O-Ⓐ in the formulas should read HES-OH and HES-O-Ⓐ, respectively, where HES and HES-O-Ⓐ mean hydroxyethyl starch and activated hydroxyethyl starch, respectively.

The above-mentioned process is now described in more detail. The reaction of activated dextran or hydroxyethyl starch with a cis-platinum(II) complex represented by the general formula (II) is generally carried out in an aqueous medium. While the reaction is generally carried out at room temperature, heating

up to about 80° C may be employed to thereby increase the reaction efficiency. The necessary reaction period may vary depending on the reaction temperature employed but generally it takes several hours to 2 days for the reaction to be complete. The activated dextran or hydroxyethyl starch and cis-platinum(II) complex are reacted with each other in an optional quantity ratio selected depending on the extent of activation of dextran or hydroxyethyl starch, the molecular weight of the cis-platinum(II) complex, the dosage form of the desired macromolecular complex as anticancer agent, and other factors but, generally, they are used in an activated dextran or hydroxyethyl starch/cis-platinum(II) conplex weight ratio within the range of about 1/0.05 to about 1/3. The use of the cis-platinum(II) complex in an increased amount results in an increased content of platinum bound to the activated dextran or hydroxyethyl starch. The desired macromolecular complex can be obtained by subjecting the reaction mixture from the activated dextran or hydroxyethyl starch and the cis-platinum(II) complex of general formula (II) directly to the separation/purification process to be mentioned later herein. For replacing the ligand of the cis-platinum(II) complex bound to the activated dextran or hydroxyethyl starch with some other ligand as required, an anion-forming compound is further added to the reaction mixture obtained in the above manner, or an anion-forming compound is added, in the presence of an aqueous solvent, to the macromolecular complex once separated from the reaction mixture and, in either case, the resulting mixture is stirred at room temperature for several hours to 1 day. As the anion-forming compound, there may be mentioned, among others, alkali metal salts, alkaline earth metal salts and ammonium salts of inorganic acids such as bicarbonic acid, carbonic acid, boric acid, sulfurous acid, nitrous acid, hydrochloric acid, sulfuric acid, nitric acid, phosphoric acid, bromic acid, hydrobromic acid, hydroiodic acid, chromic acid, manganic acid and tetraboric acid as well as alkali metal salts, alkaline earth metal salts and ammonium salts of organic acids such as acetic acid, bromoacetic acid, chloroacetic acid, trichloroacetic acid, succinic acid, maleic acid, malonic acid, oxalic acid, benzoic acid, glucuronic acid, gluconic acid, pyruvic acid, lactic acid, acetoacetic acid, methanesulfonic acid, ethylsulfuric acid, methanephosphoric acid, butylboric acid and sulfamic acids. As representatives among them, there may be mentioned sodium bicarbonate, ammonium bicarbonate, sodium chloride, potassium bromide, sodium acetate, sodium glucuronate, sodium pyruvate, sodium monochloroacetate, and sodium trichloroacetate. As hydroxy anion-forming compounds, there may be mentioned, for example, barium hydroxide, potassium hydroxide, sodium hydroxide and aqueous ammonia. The amount of the above-mentioned anion-forming compound to be added is about 1 to about 30 (preferably about 20) moles/mole of the reactant cis-platinum(II) complex of general formula (II), although said amount may vary depending on the kind of activated dextran or hydroxyethyl starch and the kind of anion-forming compound. While the time required for said anion ligand exchange varies with the kind and amount of the anion-forming compound used, it is generally in the range of several minutes to about 20 hours.

The procedure for separation and purification of the macromolecular complex from the reaction mixture thus obtained is now described in detail. Low molecular substances such as unreacted cis-platinum(II) complex and/or the excess of the anion-forming compound added for ligand exchange are removed from the reaction mixture by dialysis, gel filtration, reprecipitation, ultrafiltration, etc. The macromolecular complex-containing solution obtained after such procedure is concentrated to an appropriate extent and the concentrate is subjected to reprecipitation treatment using methanol, isopropanol or a mixed solvent composed of these, or the solution as it is lyophilized, whereby the macromolecular complex can be obtained as a solid. In the above-mentioned dialysis, ultrafiltration and gel filtration, water is used as the solvent or eluent.

As mentioned above, the macromolecular complexes according to the invention which can be obtained in the above manner have an anion and/or aqua as a ligand. It is not necessary that one and the same molecule has only one kind of ligand. The macromolecular complexes according to the invention include those having different ligands partly. The macromolecular complexes according to the invention further include those of the following types. Thus, those macromolecular complexes according to the invention which are obtained by reaction of an activated dextran or hydroxyethyl starch as activated by introduction of a polybasic acid having three or more carboxyl groups with n cis-platinum(II) complex of general formula (II), when said activated dextran or hydroxyethyl starch carried two localized carboxyl groups, for example two carboxyl groups bound to one and the same carbon atom or to two neighboring carbon atoms, include macromolecular complexes resulting from intramolecular binding of the cis-platinum(II) complex to said two carboxyl groups. For instance, those macromolecular complexes which are obtained as a result of reaction of dextran cis,cis,cis,cis-1,2,3,4-cyclopentanetetracarboxylate with a cis-platinum(II) complex of general formula (II) include macromolecular complexes of the formula given below:

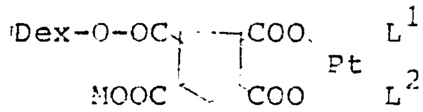

wherein $L^1$ and $L^2$ are as defined above, and M is a hydrogen atom or a salt-forming group.

The macromolecular complexes according to the invention contain the platinum atom in an amount within the range of about 0.1-20% by weight although the platinum content may vary depending on the kind of activated dextran or hydroxyethyl starch. To be capable of producing the desired pharmacological effect to a satisfactory extent and to be sufficiently soluble in water, hence readily administrable to living bodies, the macromolecular complexes should preferably have a platinum content wihtin the range of about 1-15% by weight, more preferably within the range of about 3-10% by weight. The platinum content of the macromolecular complexes can be determined by atomic absorption spectrophotometry.

The macromolecular complexes according to the invention have a molecular weight (weight-average molecular weight) of several thousand to about one million. The molecular weight of the macromolecular complexes may be selected within the above range depending on the purpose of their use. When the macromolecular complexes are administered to a tumor tissue locally or into the nutrient artery, the complexes should preferably give an appropriate degree of viscosity when dissolved in a solvent and have a tendency toward local retention. Their molecular weight is suitably within the range of 100,000-800,000, preferably within the range of 300,000-600,000. When they are administered intravenously, the macromolecular complexes suitably have a molecular weight within the range of 10,000-300,000, preferably within the range of 30,000-200,000, from the viewpoint of adequate maintenance of the blood concentration of the macromolecular ccmplexes and of reduction of their in vivo accumul ability. Whereas many of the known platinum complex anticancer agents are hardly soluble in water, the macrcmolecular complexes according to the invention, when they have a molecular weight within the range of several thousand to about 10,000, can be used for the purpose of their use merely as water-soluble anticancer agents.

## Cis-platinum (II) complexes

The cis-platinum(II) complexes of general formula (II) can be produced by a conventional method. Thus, those nitratoplatinum complexes in which $X^1$ and $X^2$ in general formula (II) are each nitrato can be obtained by reacting a cis-dichloroplatinum (II) complex of the general formula

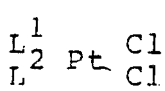

(III)

wherein $L^1$ and $L^2$ are as defined above, with 2 equivalents of silver nitrate. This reaction is generally carried out in water as a solvent at a temperature within the range of from room temperature to about 80°C. Thereafter, the resulting silver chloride precipitate is removed from the reaction mixture, and the aqueous solution obtained can be used as it is for the reaction for forming the macromolecular complexes according to the invention. This aqueous solution is acidic, when required, it can be submitted to the macromolecular complex formation reaction after pH adjustment with aqueous ammonia or aqueous sodium hydroxide solution. It is also possible to isolate the nitratoplatinum complexes as crystals by distilling off water under reduced pressure from the aqueous solution after removal of the precipitate silver chloride. In this case, the nitratoplatinum complexes obtained contain 2 molecules of water of crystallization.

The aqueous solution of nitratoplatinum complex obtained by the above method, when passed through an anion exchange resin, such as Diaion SA-10A, Amberlite IRA-400 or Dowex I, gives an alkaline aqueous solution of the corresponding hydroxoplatinum complex in which $X^1$ and $X^2$ in general formula (II) arc each hydroxo. Since such a hydroxoplatinum complex is very unstable when isolated in the solid form, the aqueous solution is submitted as it is to the reaction for forming the macromolecular complexes according to the invention.

Furthermore, when the cis-dichloroplatinum complex of general formula (III) is reacted with 1 equivalent of silver sulfate in an aqueous solvent and the resulting silver chloride is removed from the reaction mixture, there is obtained an aqueous solution of the corresponding sulfatoplatinum complex in which $X^1$ and $X^2$ in

general formula (II) combinedly represent sulfato. It is further possible to obtain an aqueous solution of the above-mentioned hydroxoplatinum complex by adding barium hydroxide to said aqueous solution of sulfatoplatinum complex and removing the resulting barium sulfate precipitate from the reaction mixture.

## Dextran

Dextran is a polysaccharide consistino of D-glucose units, linked mainly by α(1,6)pyranoglycosidic bonds. It is formed as dextran sucrase of bacterial origin acts on sucrose to thereby cleave off the fructose units of sucrose molecules. Hydrolyzates of dextran are readily soluble in water and have been used as plasma substitutes. Dextran is readily decomposed to glucose by dextranase which is widely distributed in the living body.

## Hydroxyethyl Starch

Hydroxyethyl starch is an amylopectin derivative formed as the hydrogen atoms of OH groups in amylopectin, a component unit of starch, are substituted in part by hydroxyethyl groups, and can for example be produced by permitting ethylene oxide to act on amylopectin. It is known that hydroxyethyl starch is gradually decomposed enzymatically in the body. Moreover, it is commercially available as a plasma expander. Among commercial plasma expander preparations containing hydroxyethyl starch are various infusions such as Hespander (Kyorin Pharmaceutical Co., Ltd.) and 6-H•E•S• (Morishita Pharmaceutical Co., Ltd.). Seitaizairyo, 5, 3-13, 1987 gives physicochemical and other information on hydroxyethyl starch. According to this report, the hydroxyethyl starch contained in Hespander has a weight average molecular weight of about 41,000 and a degree of hydroxyethyl substitution in the range of 0.5 to 0.55 mole (hydroxyethyl group)/mole (glucose residue) and the corresponding values of the hydroxyethyl starch contained in 6-H•E•S are about 164,000 and 0.6-0.66 mole (hydroxyethyl group)/mole (glucose residue).

## Activated Dextran and Activated Hydroxyethyl Starch

Activated dextran or activated hydroxyethyl starch is produced by reacting dextran or hydroxyethyl starch with an aliphatic di- to tetrabasic acid or a reactive derivative thereof to attach said aliphatic acid to a hydroxyl group of dextran or hydroxyethyl starch by ester bonding through one of the carboxyl groups of said acid.

When the aliphatic polybasic acid is used in the form of carboxylic acid anhydride, the reaction is conducted in a solvent such as water or dimethyl sulfoxide. When water is used as the solvent, the reaction is suitably performed in the presence of the base such as sodium bicarbonate, potassium carbonate, sodium hydroxide, bariun hydroxide or aqueous ammonia. This mode is particularly suitable for avoiding intermolecular crosslinking in carrying out the reaction using an aliphatic polybasic acid having two anhydride rings. When dimethyl sulfoxide is used as the solvent, the reaction is suitably conducted in the presence of a tertiary amine such as pyridine or triethylamine, or anhydrous sodium acetate. This mode is particularly suitable for the reaction using a polybasic acid having one anhydride ring but is not favorable for the reaction using an aliphatic polybasic acid having two anhydride rings because intermolecular crosslinking takes place. In either mode, the reaction is conducted at temperature under ice cooling to temperature under heating up to about 80 °C. Generally, however, it is convenient to perform the reaction at room tcmperature or in the vicinity thereof. A period of several hours to 1 day is sufficient for the reaction. The polybasic acid anhydride is used in an amount within the ragne of about 0.1-5 moles per monosaccharide unit in dextran or hydroxyethyl starch although the amount to be charged may vary depending on the solvent species used in carrying out the reaction. When the aliphatic polybasic acid is used as it is, the reaction is carried out in the manner of condensation in a solvent, such as dimethyl sulfoxide, in the presence of a conventional esterification catalyst with heating. For the purpose of avoiding intermolecular crosslinking, it is advisable to protect other carboxyl group or groups of the aliphatic polybasic acid than the carboxyl group to be bound to dextran or hydroxyethyl starch by a conventional method prior to submitting

said acid to reaction. It is also advisable to activate the carboxyl group to be bound to dextran or hydroxyethyl starch by converting the same to the form of acid chloride or mixed anhydride with some other carboxylic acid, for instance. As typical examples of the aliphatic polybasic acid or anhydride thereof to be used, there may be mentioned succinic acid, maleic acid, citraconic acid, glutaric acid, cis-1,2-cyclohexanedicarboxylic acid, cis-aconitic acid, 1,2,3-propanetricarboxylic acid, butane-1,2,3,4-tetracarboxylic acid, 1,1,2,3-propanetetracarboxylic acid, tetrahydrofuran-2,3,4,5-tetracarboxylic acid, 1,2,3,4-cyclopentanetetracarboxylic acid and anhydrides thereof; L-aspartic acid anhydride, L-glutamic acid anhydride; malonic acid, fumaric acid, diglycolic acid, 2,2′-thiodiglycolic aicd, 3-hydroxy-3-methylglutaric acid, (ethylenedithio)diacetic acid, N-methylglutamic acid, glutamic acid and so forth.

Carboxyl groups as introduced into dextran or hydroxyethyl starch in the above manner for activation of dextran or hydroxyethyl starch occur either in the free form or in a salt form depending on the method of dextran or hydroxyethyl starch activation. The term "activated dextran or hydroxyethyl starch" as used herein means that the carboxyl group introduced is in either of both states. Generally, from the viewpoint of stability of the activated dextran or hydroxyethyl starch and the viewpoint of reactivity with the cis-platinum(II) complex of general formula (II), it is in some instances preferable to use the activated dextran or hydroxyethyl starch in a state such that the carboxyl group introduced thereinto occurs in a salt state. When necessary, free carboxyl group-containing activated dextran or hydroxyethyl starch species are treated with a base, for example an alkali metal hydroxide such as sodium hydroxide or potassium hydroxide, an alkaline earth metal hydroxide such as calcium hydroxide or barium hydroxide, aqueous ammonia, or a tertiary amine such as pyridine, triethylamine or tri-n-butylamine, whereby the free carboxyl groups of said activated species are converted to the corresponding salt moieties.

## Utility

The macromolecular complexes according to the invention have potent anticancer activity arising from the cis-platinum(II) complex of general formula (II) as well as beneficial properties of the dextran or hydroxyethyl starch activated by an aliphatic di-, tri- or tetrabasic acid and, as such, have the following characteristic features:

The macromolecular complexes according to the invention are easily soluble in water, hence are not restricted at all with respect to the method of administration, since they have a structure such that the activated dextran or hydroxyethyl starch, which is soluble in water, and thc cis-platinun(II) complex of general formula (II) are bound to each other in the form of Dex-O-Ⓐ Pt or Hes-O Ⓐ -Pt, as mentioned hereinbefore. When administered to a living organism, the macromolecular complexes according to the invention gradually release the platinum complex in the form of an active species in the organism. As a result, an effective blood concentration of such active species is maintained for a prolonged period of time and at the same time the toxicity of said species is reduced, so that the therapeutic indices of the macromolecular complexes according to the invention are very much improved. Furthermore, the macromolecular complexes according to the invention are significantly improved with respect to such nephrotoxicity as caused by cisplatin and to such thrombocytopenia as induced by carboplatin. In addition, since solid tumors have such features as vascularization, vascular permeability and leakage from the blood vessel, the macromolecular complexes according to the invention are readily accumulated in tumor tissues without significantly affecting normal tissues. Furthermore, the macromolecular complexes according to the invention do not undergo association or crystallization. They are retained in the blood circulation system for a certain period of time and thereafter excreted through the kidney as a reuslt of in vivo metabolism of the principal chain of dextran or hydroxyethyl starch, without accumulation in tissues of living organisms. The macromolecular complexes according to the invention also show enhanced lymphatic delivery and are therefore effective in the treatment of metastasized cancer in the lymphatic system. Accordingly, the macromolecular complexes according to the invention have an extended or modified anticancer spectrum as compared with the known low-molecular-weight platinum complex anticancer agents. They have potentiated anticancer activity but do not cause severe side effects.

In the following, the results of testing in mice of the macromolecular complexes according to the invention, with cisplatin as a control, for anticancer activity against experimental tumors are shown.

### Test Example 1

Anticancer activity against mouse tumor L1210 leukemia

(1) Test by intraperitoneal administration

L1210 cells were intraperitoneally transplanted into $CDF_1$ mice (6 weeks of age, male, 6 animals/group) ($10^5$ cells/animal) and the test substances dissolved in distilled water for iniection were intraperitoneally administered one day after transplantation. The anticancer effect was evaluated in terms of the ratio, T/C (%), of the survival time in days in each treated group to the survival time in days in the untreated group. When this value was 125 % or more, the substance tested was regarded as effective. The mean survival time in the untreated group was about 8 days. Those animals that were alive on day 30 were laparotomized and examined for whether the cancer had been cured completely or not. The results thus obtained are shown in Tables 1 and 2.

The control drug cisplatin was administered as an aqueous suspension using the surfactant Tween 80.

Table 1

| Test substance | Dosage (mg/kg) | Platinum equivalent (mg/kg) | T/C* (%) | Cured mice** |
|---|---|---|---|---|
| Complex of Example 1 | 200 | 7.0 | 0 | 1/6 |
| | 100 | 3.5 | 235 | 2/6 |
| | 50 | 1.8 | 140 | |
| Complex of Example 7 | 200 | 8.6 | 183 | |
| | 150 | 6.4 | 153 | |
| Cisplatin | 12 | 7.8 | 146 | |
| | 6 | 3.9 | 128 | |
| | 3 | 2.0 | 135 | |

* T/C = 0% means that 3 or more mice died of toxicity by 5 days after transplantation.
** The number of mice with cured cancer in a group of 6 animals.

Table 2

| Test substance | Dosage (mg/kg) | Platinum equivalent (mg/kg) | T/C* (%) |
|---|---|---|---|
| Complex of Example 10 | 160<br>80<br>40 | 19.7<br>9.8<br>4.9 | 115<br>164<br>127 |
| Complex of Example 12 | 120<br>60<br>30 | 6.4<br>3.2<br>1.6 | 221<br>224<br>185 |
| Complex of Example 13 | 200<br>100<br>50 | 13.2<br>6.6<br>3.3 | 164<br>182<br>124 |
| Cisplatin | 12<br>6<br>3 | 7.8<br>3.9<br>2.0 | 143<br>131<br>136 |

* T/C = 0% means that 3 or more mice died of toxicity by 5 days after transplantation.

(2) Test by, intravenous administration

L1210 cells were intraperitoneally transplanted into $CDF_1$ mice (6 weeks of age, male, 6 animals/group) ($10^5$ cells/animal) and then the test substances dissolved in distilled water for injection were administered via the tail vein one day and five days after transplantation. The control drug cisplatin was administered as a solution in 1% aqueous mannitol solution. The anticancer effect was expressed in the same manner as in the case of testing by intraperitoneal administration as described in (1). The results obtained are shown in Table 3.

Table 3

| Test substance | Dosage (mg/kg) | Platinum equivalent (mg/kg) | T/C* (%) | Cured mice** |
|---|---|---|---|---|
| Complex of Example 2 | 250<br>125<br>62.5 | 10.3<br>5.1<br>2.6 | 0<br>329<br>195 | <br>4/6<br> |
| Complex of Example 3 | 400<br>200<br>100 | 23.6<br>11.8<br>5.9 | 79<br>177<br>133 | |
| Complex of Example 5 | 800<br>400<br>200 | 8.8<br>4.4<br>2.2 | 0<br>73<br>141 | |
| Cisplatin | 8<br>4<br>2 | 5.2<br>2.6<br>1.3 | 83<br>121<br>118 | |
| Complex of Comparative Example 1 | 100<br>50<br>25<br>12.5 | 11.0<br>5.5<br>2.8<br>1.4 | 0<br>63<br>124<br>103 | |
| Complex of Comparative Example 2 | 80<br>40<br>20 | 7.0<br>3.5<br>1.8 | 0<br>0<br>153 | |

* T/C = 0% means that 3 or more mice died of toxicity by 5 days after transplantation.

** The number of mice with cured cancer in a group of 6 animals.

Test Example 2

Effects on mouse B16 solid tumor (melanoma)

B16 melanomia cells (1 x $10^6$ cells/mouse) were subcutaneously transplanted into $BDF_1$ mice (5 weeks of age, male, 6 animals/group). On the 8th day and 12th day after transplantation, a solution of the test substance in distilled water for injection was administered to the mice through the tail vein at a dose of 0.2 ml/mouse. During the period from the 8th day to 24th day after transplantation, the tumor size was determined with a caliper gauge at intervals of 3-4 days and the tumor weight was estimated using the formula:

Estimated tumor weight = $\frac{1}{2}$ x (minor axis of tumor)$^2$ x (major axis of tumor)

The results thus obtained are shown in Figs. 13 and 14. In these figures, the ordinate indicates the relative tumor weight (the ratio of an estimated tumor weight up to day 24 to the estimated tumor weight on day 8). The lines (a), (b) and (c) in Fig. 13 represent changes in relative tumor weight after transplantation in the control group, Example 2 complex 180 mg/kg/dose group and cisplatin 3 mg/kg/dose group, respectively. The lines (a), (b) and (c) in Fig. 14 represent changes in relative tumor weight after transplantation in the control group, Example 12 complex 125 mg/kg/dose group and cisplatin 3 mg/kg/dose group.

It will be apparent from Tables 1 through 3 that the macromolecular complexes of the invention display marked anticancer activity which is definitely higher than that of the control drug cisplatin. Furthermore, some of the macromolecular complexes of the invention cure cancers at a high efficacy rate. It is also

apparent from Fig. 13 and 14 that the macromolecular complexes of this invention have greater antitumor activity than cisplatin against solid tumors and that they cause no loss of body weight in mice even at the dose used in Test Example 2, thus being very low in toxicity. In contrast, cisplatin at the dose of Test Example 2 or higher causes a marked decrease in body weight which ultimately leads to death. In addition, the macromolecular complexes of the invention are water-soluble and are easy to formulate into various dosage forms. The complexes according to Comparative Examples 1 and 2 develop intense toxicity and are inferior to the macromolecular complexes in anticancer activity of this invention.

As explained hereinabove, the macromolecular complexes according to the invention are useful as therapeutic agents for cancer in mammals such as horses, cattle, pigs, dogs, mice, rats, guinea-pigs and, among others, humans.

The macromolecular complexes according to the invention can release low-molecular platinum complexes, which are active anticancer species, even under mild conditions and it is presumable that they release such active species as the corresponding platinum monochlorides or dichlorides complex as a result of inter action with, for example, sodium chloride in living organisms. When interactions with various biological components in living organisms are taken into consideration, however, the cleavage of the macromolecular complexes is by no means simple. In any way, the macromolecular complexes according to the invention have various characteristic features, as mentioned above, when compared with the known low-molecular platinum complex anticancer agents, and can be used as novel and excellent macromolecular anticancer agents.

The effective dose of the macromolecular complexes according to the invention can be varied within a broad range depending on various factors such as platinum content in the macromolecular complexes, method of administraiton, symptoms of the patient to be treated, properties of the cancer to be treated, body weight, age, sex, and judgement of the physician in charge of treatment. In the case of humans, the dose can generally be within the range of about 0.3-20 g/day as expressed in terms of the macromolecular complexes and such dose can be administered in a single dose or several divided doses daily.

The macromolecular complexes according to the invention can be administered to humans and other mammals such as horses, cattle, pigs, dogs, mice, rats and guinea pigs either orally or nonorally. Generally, treatment by nonoral administration is preferred. The macromolecular complexes according to the invention give good results when they are dissolved or suspended in an appropriate solvent for injection, such as distilled water for injection, 1% aqueous sodium bicarbonate solution, 5% aqueous glucose solution, ethanol-containing water, glycerol-containing water or propylene glycol-containing water, and administered by intravenous injection, intramuscular injection, subcutaneous injection or drip infusion, for instance. When required, they can be injected intraarterially or locally into cancer tissues as well. In the latter case, the macromolecular complexes according to the invention, which are macromolecular substances, are apt to be retained locally and release active species gradually over a prolonged period of time, whereby the excellent anticancer effects of the macromolecular complexes of the invention are produced.

Accordingly, the present invention also includes pharmaceutical compositions containing at least one of the above-mentioned macromolecular complexes.

The macromolecular complexes according to the invention can be used in combination of one or more other anticancer agents and, furthermore, can be formulated in combination with various drugs, such as buffers, local anesthetics, hypnotics and analgesics, for providing desired pharmacological properties. While the macromolecular complexes according to the invention can be stored in ampuls in the form of a solution or suspension, it is preferable to store them in ampuls or vials in the form of a powder or lyophilizate and reconstitute an appropriate dosage form prior to use.

The following examples are intended to illustrate this invention in futher detail and should by no means be construed as limiting the scope of the invnetion.

## Reference Example 1

In 20 ml of dimethyl sulfoxide was dissolved 2.0 g of dextran 70 (Japanese Pharmacopoeia, average molecular weight ca. 70,000) followed by addition of 3.2 g of pyridine. Then, a solution of 0.4 g of maleic anhydride in 12 ml of dimethyl sulfoxide was added dropwise to the above solution with constant stirring. After completion of dropwise addition, the mixture was stirred at room temperature overnight. This reaction mixture was added dropwise to about 3 ℓ of isopropyl alcohol for reprecipitation. The resulting precipitate was dissolved in about 15 ml of water and the solution was subjected again to reprecipitation with about 3 ℓ of isopropyl alcohol. The procedure gave 2.1 g of dextran maleic half-ester.

## Reference Example 2

The procedure of Reference Example 1 was repeated except that 0.4 g of succinic anhydride was used in lieu of 0.4 g of maleic anhydride to give 1.8 g of dextran succinic half-ester.

## Reference Example 3

In 20 ml of aqueous sodium hydrogen carbonate solution (0.8 mole/liter) was dissolved 2.0 g of dextran 70 and while the solution was maintained at pH 7 or higher by gradual addition of 12.0 g of sodium hydrogen carbonate powder, 5.6 g of cis, cis, cis, cis-1,2,3,4-cyclopentanetetracarboxylic dianhydride was added gradually over 10 hours. The mixture was then stirred at room temperature overnight. Using an ultrafiltration membrane (Sartorius, cutoff molecular weight 20,000), the reaction mixture was filtered for one day and the filtrate was lyophilized to give 1.8 g of dextran mono-cis, cis, cis, cis-1,2,3,4-cyclopentanetetracarboxylic half-ester sodium salt.

## Reference Example 4

In 20 ml of aqueous sodium hydrogen carbonate solution (0.8 mole/liter) was dissolved 2.0 g of dextran with a molecular weight of 2900 ( $\overline{M}w/\overline{M}n$ = 1.66) and while the solution was maintained at pH7 or higher by addition of 12.0 g of sodium hydrogen carbonate powder, 5.7 g of cis, cis, cis, cis-1,2,3,4-cyclopentanetetracarboxylic acid dianhydride was gradually added over 8 hours. The mixture was then stirred at room temperature overnight. Thc resulting reaction mixture was subjected to gel permeation chromatography (solid phase: Sephadex G-25, Pharmacia Fine Chemicals, eluent: distilled water) and the high molecular fractions were pooled and lyophilized to give 1.8 g of dextran mono-cis, cis, cis, cis-1,2,3,4-cyclopentanetetracarboxylic half-ester sodium salt.

## Reference Example 5

The procedure of Reference Example 3 was repeated except that dextran 40 (J. P., average molecular weight about 40,000) and 5.8 g of tetrahydrofuran-2,3,4,5,-tetracarboxylic dianhydride were used in lieu of dextran 70 and 5.6 g of cis, cis, cis, cis-1,2,3,4-cyclopentanetetracarboxylic dianhydride, respectively. The procedure gave 1.9 g of dcxtran mono-tetrahydrofuran-2,3,4,5-tetracarboxylic half-ester sodium salt.

## Reference Example 6

A commercial infusion containing 6% of hydroxymethyl starch (Hespander, Kyorin Pharmaceutical Co., Ltd) (300 ml) was lyophilized and the resulting solid was dissolved in about 100 ml of water. The solution was reprecipitated from 4 ℓ of isopropyl alcohol to give 17 g of hydroxyethyl starch.

## Reference Example 7

In 30 ml of dimethyl sulfoxide was dissolved 3.0 g of hydroxyethyl starch obtained in the same manner as Reference Example 6 and, then, 4.7 g of pyridine was added to the solution. Thereafter, a solution of 0.7 g of maleic anhydride in 18 ml of dimethyl sulfoxide was added dropwise to the above solution over a period of about 20 minutes with constant stirring. After completion of dropwise addition, the mixture was

stirred at room temperature for 6 hours. This reaction mixture was added dropwise to about 3 ℓ of isopropyl alcohol for reprecipitation. The precipitate was dissolved in 60 ml of water and the solution was dialyzed using a dialysis membrane (tube sixe 30/32, Union Carbide Corp.) for 2 days. Finally, the dialyzate was lyophilized to give 1.8 g of hydroxyethyl starch maleic half-ester.

## Reference Example 8

In 25 ml of dimethyl sulfoxide was dissolved 3.0 g of hydroxyethyl starch obtained in Reference Example 6, followed by addition of 4.7 g of pyridine. Then, a solution of 0.7 g of succinic anhydride in 10 ml of dimethyl sulfoxide was added dropwise to the above solution over a period of about 20 minutes with constant stirring. After completion of dropwise addition, the mixture was stirred at room temperature for 6 hours. This reaction mixture was reprecipitated from 2 ℓ of isopropyl alcohol. The precipitate was dissolved in 15 ml of water and the solution was reprecipitated again from 2 ℓ of isopropyl alcohol. The above procedure gave 2.0 g of hydroxyethyl starch succinic half-ester.

## Reference Example 9

In 20 ml of aqueous sodium hydrogen carbonate solution (0.8 mole/liter) was dissolved 2.0 g of hydroxyethyl starch obtained in the same manner as Reference Example 6 and while the solution was maintained at pH 7 or higher by gradual addition of 6.0 g of sodium hydrogen carbonate powder, 2.8 g of cis, cis, cis, cis-1,2,3,4-cyclopentanetetracarboxylic dianhydride was gradually added over 10 hours. The mixture was then stirred at room temperature overnight. Thereafter, with 6.0 g of sodium hydrogen carbonate being added gradually to the reaction mixture, 2.8 g of cis, cis, cis, cis-1,2,3,4-cyclopentanetetracarboxylic dianhydride was added over a period of 3 hours. The mixture was further stirred at room temperature for 3 hours. Using an ultrafiltration membrane (Sartorius, SM14539; cutoff molecular weight 10,000), the reaction mixture was filtered for 10 hours and the filtrate was dialyzed using a dialysis membrane (tube size 30/32, Union Carbide Corp.) for 2 days. Finally the dialyzate was lyophilized to give 1.3 g of hydroxyethyl starch mono-cis, cis, cis, cis-1,2,3,4-cyclopentanetetracarboxylic half-ester sodium salt.

## Reference Example 10

In 160 ml of water was dissolved 15.00 g of potassium chloroplatinoate and a solution of 4.20 g of trans-1,2-diaminocyclohexane (abbreviated as DACH; Aldrich Chemical Co.) in 20 ml of water was added to the above solution. The mixture was stirred at room temperature for one day and the resulting crystals were recovered by filtration and recrystallized from about 25 ℓ of 0.1N hydrochloric acid. The procedure gave 8.80 g of cis-dichloro(DACH)platinum (II) as yellow needles.

To a mixture of 388 mg (1.02 mmoles) of the above cis-dichloro(DACH)platinum (II) and 6 ml of water was added a solution of 340 mg (2.00 mmoles) of silver nitrate in 3 ml of water and the mixture was stirred in the dark at about 60 °C for 40 minutes. Then, the mixture was further stirred at room temperature overnight. Thereafter, the precipitated silver chloride was filtered off to give an aqueous solution of cis-dinitrato(DACH)platinum (II).

## Reference Example 11

In 180 ml of water was dissolved 15.00 g of potassium chloroplatinoate, followed by addition of 15.00 g of ammonium chloride. The mixture was stirred. To the resulting solution was added 28.0 ml of 3N aqueous ammonia and the mixture was stirred in the dark at 3 °C or less for 5 days. The resulting crystals were recovered by filtration and dissolved in 500 ml of N,N-dimethylformamide. The insoluble matter was filtered off. The filtrate was added dropwise to 1 ℓ of methanol and the mixture was stirred at room temperature for

2 hours. The crystals which separated out were collected by filtration and recrystallized from 430 ml of 0.1N hydrochloric acid. The procedure gave 6.13 g of cis-dichlorodiammineplatinun (II) as yellow crystals.

To a mixture of 122 mg (0.41 mmole) of cis-dichlorodiammineplatinum (II) and 5 ml of water was added a solution of 136 mg (0.80 mmole) of silver nitrate in 3 ml of water and the mixture was stirred at about 60°C in the dark for 1 hour and, then, at room temperature overnight. Thereafter, the precipitated silver chloride was filtered off. The procedure gave an aqueous solution (filtrate) of cis-dinitratodiammineplatinum (II).

## Reference Example 12

In 65 ml of water was dissolved 6.23 g of potassium chloroplatinoate followed by addition of a solution of 1.80 g of 2-aminomethylpyridine in 10 ml of water. The mixture was stirred at room temperature for one day. The resulting crystals were collected by filtration, rinsed and dried. The procedure gave 4.39 g of cis-dichloro(2-aminomethylpyridine)platinum (II) as yellow crystals.

To a mixture of 194 mo (0.52 mmole) of the above cis-dichloro(2-aminomethylpyridine)platinum (II) and 5 ml of water was added a solution of 136 mg (0.80 mmole) of silver nitrate in 2 ml of water and the mixture was stirred in the dark at about 60°C for 40 minutes and, then, at room temperature overnight. Thereafter, the precipitated silver chloride was filtcred off. The procedure gave an aqueous solution (filtrate) of cis-dinitrato(2-aminomethylpyridine)platinum (II).

## Reference Example 13

In 130 ml of water was dissolved 3.79 g of potassium chloroplatinoate and a solution of 6.07 g of potassium iodide in 20 ml of water was added to the above solution with co::sta:t stirring. The mixture was further stirred at 35°C for 10 minutes. A flask was filled with 95 ml of water and, under stirring at 60°C, the above-prepared aqueous solution of potassium iodoplatinoate and a solution of 0.93 g of 2-methyl-1,4-butanediamine in 250 ml of water were simultaneously added dropwise over 3 hours. The resulting tan-colored precipitate was recovered by filtration and rinsed and the powder thus obtained was dried under reduced pressure. The procedure gave 3.57 g of cis-diiodo(2-methyl-1,4-butanediamine)platinum.

To a mixture of 562 mg (1.02 mmoles) of the above cis-diiodo(2-methyl-1,4-butanediamine)platinun (II) and 8 ml of water was added a solution of 340 mg (2.00 mmoles) of silver nitrate in 4 ml of water and the mixture was stirred in the dark at about 60°C for 40 minutes and, then, at room temperature overnight. Thereafter, the precipitated silver iodide was filtered off. The procedure gave an aqueous solution (filtrate) of cis- dinitrato(2-methyl-1,4-butanediamine)platinum (II).

## Reference Example 14

In 100 ml of water was dissolved 4.15 g of potassium chloroplatinoate and a solution of 1.00 g of 2-aminomethylpyrrolidine in 10 ml of water was added. The mixture was stirred at room temperature for one day. The resulting crystals were collected by filtration, rinsed and dried. The procedure gave 3.03 g of cis-dichloro(2-aminomethylpyrrolidine)platinum (II) as yellow crystals.

To a mixture of 373 mg (1.02 mmoles) of the above cis-dichloro(2-aminomethylpyrrolidine)platinum (II) and 30 ml of water was added a solution of 340 mg (2.00 mmoles) of silver nitrate in 3 ml of water and the mixture was stirred at room temperature in the dark for 3 days. Finally the precipitated silver chloride was filtered off. The procedure gave an aqueous solution (filtrate) of cis-dinitrato(2-aminomethylpyrrolidine)-platinum (II).

## Example 1

In 40 ml of water was dissolved 1000 mg of the dextran succinic half-ester sodium salt obtained in Reference Example 2. Then, an aqueous solution of cis-dinitrato(DACH)platinum (II) as prepared in accordance with Reference Example 10 was added thereto and the mixture was stirred at room temperature in the dark overnight. The reaction mixture was subjected to gel permeation chromatography (solid phase: Sephadex G-25, Pharmacia Fine Chemicals; eluent: distilled water) and the high molecular fractions were pooled and lyophilized. This procedure gave 980 mg of cis-nitrato- and aqua(DACH)platinum (II)-dextran succinic half-ester complex [actually, macromolecular complexes formed on partial binding of cis-nitrato-(DACH)platinum (II) and the corresponding aqua (DACH)platinum (II) to dextran succinic half-ester and the complex of general formula (I′-1) wherein $L^1$ and $L^2$ combinedly represent trans-1,2-diaminocyclohexane; $X^3$ is a nitrato ligand group, and Dex-O-Ⓐ represents dextran succinic half-ester and the complex of general formula (I′-2) wherein $L^1$ and $L^2$ combinedly represent trans-1,2-diaminocyclohexane; $X^3$ is a nitrato ligand group and Dex-O-Ⓐ represents dextran succinic half-ester: the same applies hereinafter). The platinum content of this macromolecular complex was 3.5 weight %. The infrared absorption spectrum of this complex is presented in Fig. 1.

## Example 2

In 40 ml of water was dissolved 1000 mg of the dextran mono-cis, cis, cis, cis-1,2,3,4-cyclopentanetetracarboxylic half-ester sodium salt obtained in Reference Example 3. To this solution was added an aqueous solution of cis-dinitrato(DACH)platinum (II) as prepared according to Reference Example 10 and the mixture was stirred at room temperature in the dark overnight. The reaction mixture was dialyzed using a dialysis membrane (tube size 30/32, Union Carbide Corp.) for 2 days and the dialyzate was lyophilized. The procedure gave 760 mg of cis-nitrato- and aqua(DACH)platinum (II)-dextran mono-cis, cis, cis, cis-1,2,3,4-cyclopentanetetracarboxylic half-ester complex, and cis(DACH)platinum (II)-dextran mono-cis, cis, cis, cis-1,2,3,4-cyclopentanetetracarboxylic half-ester complex [the macromolecular complex formed as two bonding sites of cis-(DACH)platinum (II) are bound to the residues available on removal of one hydrogen atom each from the two carboxyl groups attached to the neighboring carbon atoms of dextran mono-cis, cis, cis, cis-1,2,3,4-cyclopentanetetracarboxylic half-ester: which is the complex of general formula (I-3) wherein $L^1$ and $L^2$ combinedly represent trans-1,2-diaminocyclohexane and Dex-O-Ⓐ represents dextran mono-cis, cis, cis, cis- 1,2,3,4-cyclopentanetetracarboxylic half-ester: the same applies hereinafter]. The platinum content of this macromolecular complex was 4.1 weight %. The infrared absorption spectrum (FT-IR) of the complex is presented in Fig. 2.

## Examples 3 to 5

In 40 ml of water was dissolved 1000 mg of one of activated dextrans which had been prepared according to Reference Examples (Table 4) to give an aqueous solution. To each of these aqueous solutions was added an aqueous solution of cis-dinitrato(DACH) platinum (II) as prepared according to Reference Example 10 and the mixture was stirred at room temperature in the dark overnight. To this reaction mixture was added a predetermined quantity of an anion-forming compound and the mixture was stirred at room temperature. When sodium hydrogen carbonate was used as the anion-forming compound, the reaction mixture was stirred at room temperature overnight. When sodium chloride was used as the anion-forming compound, the reaction mixture was warmed at about 40 to 60 °C for several minutes and, then, stirred at room temperature overnight. Then, the precipitate was removed by centrifugation and the supernatant was subjected to gel permeation chromatography (solid phase: Sephadex G-25, Pharmacia Fine Chemicals; eluent distilled water). The high molecular fractions were pooled and lyophilized to give the desired macromolecular complex. The yields and platinum contents of the complexes obtained in the above manner are shown in Table 4. Though the Product complex column of the table gives a listing of macromolecular complexes of predominant structures, the respective complexes include structures containing nitrato ligand groups. The infrared absorption spectra (FT-IR) of the macromolecular complexes according to Examples 3 to 5 are shown in Figs. 3 to 5, respectively.

Table 4

| Example No. | Activated dextran (Reference Example) | Anion-forming compound mg (mmole) | Product complex<br>Yield (mg)<br>Pt content (wt. %) |
|---|---|---|---|
| 3 | Dextran maleic half-ester (Ref. Ex. 1) | $NaHCO_3$<br>700(8.3) | cis-Hydrogencarbonato[and aqua](DACH)platinum (II)-dextran maleic half-ester complex<br>750<br>Pt 5.9 |
| 4 | Dextran succinic half-ester (Ref. Ex. 2) | NaCl<br>117(2.0) | cis-Chloro[and aqua](DACH)platinum (II)-dextran succinic half-ester complex<br>680<br>Pt 1.1 |
| 5 | Dextran mono-tetra-hydrofuran-2,3,4,5-tetracarboxylic half-ester sodium salt (Ref. Ex. 5) | $NaHCO_3$<br>340(4.0) | cis-Hydrogencarbonato[and aqua](DACH)platinum (II)-dextran mono-tetrahydrofuran-2,3,4,5-tetracarboxylic half-ester complex and cis-(DACH)platinum (II)-dextran mono-tetrahydrofuran-2,3,4,5-tetracarboxylic half-ester complex<br>700<br>Pt 1.6 |

Examples 6 to 8

In 12 ml of water was dissolved 400 mg of one of activated dextrans as prepared according to the Reference Examples mentioned in Table 5 to give an aqueous solution of activated dextran. To each of these aqueous activated dextran solutions was added an aqueous solution of the platinum complex prepared according to the Reference Example mentioned in Table 5, and the mixture was stirred at room temperature in the dark overnight. The reaction mixture was dialyzed using a dialysis membrane (tube size 18/32, Union Carbide Corp.) for 2 days and the dialyzate was filtered to remove the small quantity of insoluble matter. The filtrate was then lyophilized to recover the desired macromolecular complex. The yields and platinum contents of these complexes are shown in Table 5. The infrared absorption spectra (FT-IR) of the complexes according to Examples 6 and 7 are presented in Figs. 6 and 7, respectively.

Table 5

| Example No. | Activated dextran (Reference Example) | Starting aqueous platinum complex solution mg (mmole) (Reference Example) | Product complex Yield (mg) Pt content (wt. %) |
|---|---|---|---|
| 6 | Dextran maleic half-ester (Ref. Ex. 1) | cis-Dinitrato(2-amino-methylpyridine)platinum (II) (Ref. Ex. 12) | cis-Nitrato[and aqua](2-aminomethyl-pyridine)platinum (II)-dextran maleic half-ester complex<br>300<br>Pt 5.2 |
| 7 | Dextran succinic half-ester (Ref. Ex. 2) | cis-Dinitratodiamine-platinum (II) (Ref. Ex. 11) | cis-Nitrato[and aqua]diammineplatinum (II)-dextran succinic half-ester complex<br>350<br>Pt 4.3 |
| 8 | Dextran mono-cis, cis, cis, cis-1,2,3,4-cyclopentanetetracarboxylic half-ester sodium salt (Ref. Ex. 4) | cis-Dinitrato(2-metyl-1,4-butanediamine)-platinum (II) (Ref. Ex. 13) | cis-Nitrato[and aqua](2-methyl-1,4-butanediamine)platinum (II)-dextran-mono-cis, cis, cis, cis-1,2,3,4-cyclo pentanetetracarboxylic half-ester complex, and cis-(2-methyl-1,4-butane-diamine)platinum (II)-dextrane-mono-cis, cis, cis, cis-1,2,3,4-cyclopentanetetra-carboxylic half-ester complex<br>340<br>Pt 4.8 |

## Example 9

The procedure of Example 1 was repeated except that the dextran mono-cis, cis, cis, cis-1,2,3,4-cyclopentanetetracarboxylic half-ester sodium salt obtained in Reference Example 4 was used in lieu of dextran succinic half-ester sodium salt to give 650 mg of cis-nitrato[and aqua](DACH)platinum (II)-dextran mono-cis, cis, cis, cis-1,2,3,4-cyclopentanetetracarboxylic half-ester complex and cis-(DACH)platinum (II)-dextran mono-cis, cis, cis, cis-1,2,3,4-cyclopentanetetracarboxylic half-ester complex. The platinum content of the above macromolecular complex was 7.1 weight %. The infrared absorption spectrum (FT-IR) of the complex is shown in Fig. 8.

## Examples 10-12

In a predetermined quantity of water was dissolved 1000 mg of one of activated hydroxyethyl starches as prepared according to the Reference Examples mentioned in Table 6. To each of these aqueous solutions was added an aqueous solution of cis-dinitrato(DACH)platinum (II) obtained in Reference Example 10 and the mixture was stirred at room temperature in the dark overnight. Using a dialysis membrane (tube size 30/32, Union Carbide Corp.), tho reaction mixture was dialyzed for 2 days and after the insoluble matter, if any, was filtered off, the dialyzate was lyophilized to recover the desired complex.

The cis-(DACH)platinum (II)-hydroxyethyl starch mono-cis, cis, cis, cis-1,2,3,4-cyclopentanetetracarboxylic half-ester complex obtained in Example 12 is a complex formed as the two binding sites of cis-(DACH)-platinum (II) are bound to the residues available on removal of one hydrogen atom each from the two

$-\overset{\overset{O}{\|}}{C}-OH$ groups at the neighboring carbon atoms of hydroxyethyl starch mono-cis, cis, cis, cis-1,2,3,4-cyclopentanetetracarboxylic half-ester. Referring to the general formula (I-3), the above macromolecular complex corresponds to the complex of the formula

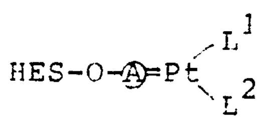

wherein $L^1$ and $L^2$ combinedly represent 1,2-diaminocyclohexane and Ⓐ represents

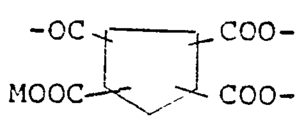

(where M means a hydrogen atom or a salt-forming group). The yields and platinum contents of these macromolecular complexes are shown in Table 6. The infrared absorption spectra (FT-IR) of the complexes according to Examples 10-12 are presented in Figs. 9-11, respectively.

Table 6

| Example No. | Activated hydroxyethyl starch (Reference Example) | Quantity of water (ml) | Product complex<br>Yield (mg)<br>Pt content (wt. %) |
|---|---|---|---|
| 10 | Hydroxyethyl starch maleic half-ester (Ref. Ex. 7) | 50 | cis-Nitrato[and aqua](DACH)platinum (II)-hydroxyethyl starch maleic half-ester complex<br>1000<br>Pt 12.3 |
| 11 | Hydroxyetyl starch succinic half-ester (Ref. Ex. 8) | 40 | cis-Nitrato[and aqua](DACH)platinum (II)-hydroxyethyl starch succinic half-ester complex<br>890<br>Pt 9.1 |
| 12 | Hydroxyethyl starch mono-cis, cis, cis, cis-1,2,3,4-cyclopentanetetracarboxylic half-ester sodium salt (Ref. Ex. 9) | 70 | cis-Nitrato[and aqua](DACH)platinum (II)-hydroxyethyl starch mono-cis, cis, cis, cis-1,2,3,4-cyclopentanetetracarboxylic half-ester complex and cis-(DACH)platinum (II)-hydroxyethyl starch mono-cis, cis, cis, cis-1,2,3,4-cyclopentanecarboxylic half-ester complex<br>1000<br>Pt 5.3 |

Examples 13 to 15

The procedures of Examples 3 to 5 were repeated except that 1000 mg of activated hydroxyethyl starches as obtained according to the Reference Examples mentioned in Table 7 were used in lieu of 1000 mg of activated dextran to give the corresponding complexes. The yields and platinum contents of these macromolecular complexes are given in Table 7. The infrared absorption spectrum (FT-IR) of the complex according to Example 13 is presented in Fig. 12.

Table 7

| Example No. | Activated hydroxyethyl starch (Reference Example) | Anion-forming compound mg (mmoles) | Product complex<br>Yield (mg)<br>Pt content (wt. %) |
|---|---|---|---|
| 13 | Hydroxyethyl starch maleic half-ester (Ref. Ex. 7) | $NaHCO_3$<br>700(8.3) | cis-Hydrogencarbonato[and aqua](DACH)platinum (II)-hydroxyethyl starch maleic half-ester complex<br>810<br>Pt 6.6 |
| 14 | Hydroxyethyl starch succinic half-ester (Ref. Ex. 8) | NaCl<br>117(2.0) | cis-Chloro[and aqua](DACH)platinum (II)-hydroxyethyl starch succinic half-ester complex<br>740<br>Pt 2.4 |
| 15 | Hydroxyethyl starch maleic half-ester (Ref. Ex. 7) | $CH_3COONa \cdot 3H_20$<br>540(4.0) | cis-Acetato[and aqua](DACH)platinum (II)-hydroxyethyl starch maleic half-ester complex<br>760<br>Pt 4.1 |

## Example 16

In 40 ml of water was dissolved 1000 mg of hydroxyethyl starch maleic half-ester as prepared according to Reference Example 7, followed by addition of an aqueous solution of cis-dinitrato(2-aminomethylpyrrolidine)platinum (II) as prepared according to Reference Example 14 and the mixture was stirred in the dark at room temperature overnight. Thereafter, the reaction mixture was worked up in the same manner as Example 1 to give 860 mg of cis-nitrato[and aqua] (2-aminomethylpyrrolidine)platinum (II)-hydroxyethyl starch maleic half-ester complex. The platinum content of this macromolecular complex was 6.9 weight %.

## Comparative Example 1

In 25 ml of a 30% aqueous solution of sodium hydroxide was dissolved 5.0 g of dextran 70 under heating. While this solution was maintained at a temperature of 70-80° C, 6.0 g of monochloroacetic acid was added thereto in several portions and the mixture was stirred for 4 hours. This reaction mixture was subjected to reprecipitation using about 5 ℓ of methanol. The resulting precipitate was dissolved in about 30 ml of water and the solution was subjected again to reprecipitation using about 5 ℓ of methanol. The precipitate thus obtained was dissolved in about 500 ml of water and using a dialysis membrane (tube size 30/32, Union Carbide Corp.), the solution was dialyzed for 2 days. Finally the dialyzate was lyophilized to give 4.7 g of carboxymethyl dextran sodium salt.

In 12 ml of water was dissolved 400 mg of the above carboxymethyl dextran sodium salt, followed by addition of an aqueous solution of cis-dinitratodiammineplatinum (II) as prepared according to Reference Example 11, and the mixture was stirred in the dark at room temperature overnight. Using a dialysis membrane (tube size 18/32, Union Carbide Corp.), this reaction mixture was dialyzed for 2 days and the dialyzate was lyophilized. The procedure gave 370 mg of cis-nitrato[and aqua]diammineplatinum (II)-carboxymethyl dextran complex. The platinum content of this complex was 11.0 weight %.

## Comparative Example 2

In 40 ml of water was dissolved 1000 mg of dextran sulfate sodium salt (Sigma, mol. wt. about 5000) and the resulting solution was added to an aqueous solution of cis-dinitrato(DACH)platinum (II) as prepared according to Reference Example 10. The mixture was stirred in the dark at room temperature overnight. Using a dialysis membrane (tube size 30/32, Union Carbide Corp.), the reaction mixture was dialyzed for 1 day and the dialyzate (internal fluid) was lyophilized to gave 1180 mg of cis-nitrato[and aqua](DACH)-platinum (II)-dextran sulfate complex. The platinum content of this complex was 8.8 wt. % and the sulfur content thereof was 13.0 wt. %.

When allowed to stand at room temperature for many hours, the above complex turned brown and syrupy, thus being found to be unstable.

## Comparative Example 3

Carboxymethylcellulose sodium was synthesized by the process described at 216 page in Kobunshi Kagaku Jikkenho (Experimental Methods in Polymer Chemistry) (translated and edited by Yoshio Iwakura, Asakura Shoten, published 1968). Thus, 15.0 g of cellulose powder was added to 400 ml of isopropyl alcohol and the mixture was shaken vigorously in a nitrogen gas stream. Then, 50 g of 30% aqueous sodium hydroxide solution was added over 15 minutes and the mixture was further stirred for 30 minutes. A solution of 17.5 g of monochloroacetic acid in 50 ml of isopropyl alcohol was added to the above reaction mixture over a period of 30 minutes, and the mixture was further stirred at about 60° C for 4 hours.

The reaction mixture was neutralized with glacial acetic acid using phenolphthalein as an indicator and,

then, filtered. The resulting solid product was dispersed in 400 ml of 80% aqueous methanol at a temperature of about 60 ° C and the dispersion was filtered and the residue was washed with methanol containing a small amount of water. The above procedure of dispersion into aqueous methanol, filtration and washing was repeated 3 times and the product was finally washed with pure methanol and dried in vacuo at a temperature of about 50 ° C. The procedure gave 22.8 g of powdery carboxymethylcellulose sodium salt.

In 60 ml of water was dissolved 1000 mg of the carboxymethylcellulose sodium powdcr thus obtained and the solution was added dropwise to an aqueous solution of cis-dinitrato(DACH)platinum (II) as prepared according to Reference Example 10, with constant stirring at room temperature. However, the reaction mixture underwent thorough gelation before completion of dropwise addition. Thus, the desired water-soluble complex could not be obtained.

When, in the above synthesis of carboxymethylcellulose sodium, the degree of carboxymethyl substitution was decreased by halving the feed of monochloroacetic acid, the resulting product was soluble in water only by about 50%.

## Advantages of the Invention

The macromolecular complexes provided in accord ance with this invention are water-soluble and as apparent from the test results described hereinbefore, have excellent anticancer activity and are low in toxicity. Anticancer preparations containing these complexes as active ingredients assure an effective development of the excellent anticancer activity possessed by the complexes.

## Claims

1. A dextran derivative having one or more carboxylated active sites resulting from reaction with an aliphatic di- to tetrabasic acid or a reactive derivative thereof, wherein one hydrogen atom of the group of the formula

$$-\overset{\overset{O}{\|}}{C}-OH$$

occurring at said active sites is partly substituted by a group of the general formula

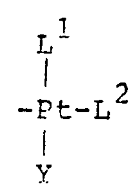

or

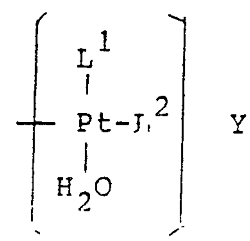

wherein L¹ and L² each independently is ammine or a unidentate ligand amine or combinedly represent a bidentate ligand amine and Y is an anionic ligand, and/or two hydrogen atoms of two $-\overset{\overset{O}{\|}}{C}-OH$ groups bound to one and the same carbon atom or to two neighboring carbon atoms as occurring at said active sites are, each independently, partly substituted by a group of the general formula

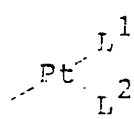

wherein $L^1$ and $L^2$ are as defined above, or a pharmacologically acceptable salt thereof.

2. A hydroxyethyl starch derivative having one or more carboxylated active sites resulting from reaction with an aliphatic di- to tetrabasic acid or a reactive derivative thereof, wherein one hydrogen atom of the group of the formula

$-\overset{O}{\overset{\|}{C}}-OH$
occurring at said active sites is partly substituted by a group of the general formula

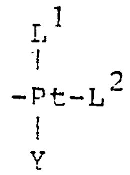 or 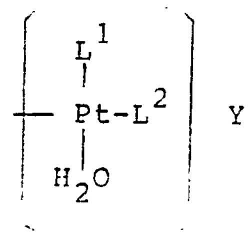

wherein $L^1$ and $L^2$ each independently is ammine or a unidentate ligand amine or combinedly represent a bidentate ligand amine and Y is an anionic ligand, and/or two hydrogen atoms of two $-\overset{O}{\overset{\|}{C}}-OH$ groups bound to one and the same carbon atom or to two neighboring carbon atoms as occurring at said active sites are, each independently, partly substituted by a group of the general formula

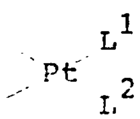

wherein $L^1$ and $L^2$ are as defined above, or a pharmacologically acceptable salt thereof.

3. A process for producing a dextran derivative having one or more carboxylated active sites resulting from reaction with an aliphatic di- to tetrabasic acid or a reactive derivative thereof, wherein one hydrogen atom of the group of the formula
$-\overset{O}{\overset{\|}{C}}-OH$
occurring at said active sites is partly substituted by a group of the general formula

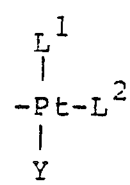 or 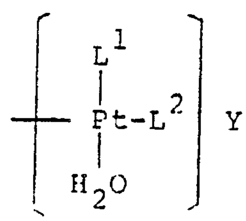

wherein $L^1$ and $L^2$ each independently is ammine or a unidentate ligand amine or combinedly represent a bidentate ligand amine and Y is an anionic ligand, and/or two hydrogen atoms of two $-\overset{O}{\overset{\|}{C}}-OH$ groups bound to one and the same carbon atom or to two neighboring carbon atoms as occurring at said active sites are, each independently, partly substituted by a group of the general formula

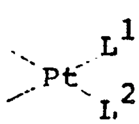

wherein $L^1$ and $L^2$ are as defined above, and/or a pharmacologically acceptable salt thereof which comprises reacting dextran with an aliphatic di-, tri- or tetrabasic acid or a reactive derivative thereof, then reacting the thus-obtained activated dextran with a cis-platinum(II) complex of the general formula

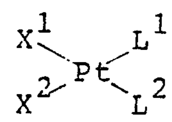

wherein L' and L² are as defined above and $X^1$ and $X^2$ each is nitrato or hydroxo or combinedly represent sulfato, to obtain a dextran derivative having one or more carboxylated active sites resulting from reaction with an aliphatic di- to tetrabasic acid or a reactive derivative thereof, wherein one hydrogen atom of the group of the formula

$-\overset{\overset{O}{\|}}{C}-OH$

occurring at said active sites is partly substituted by a group of the general formula

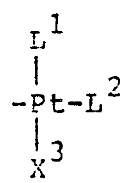 or 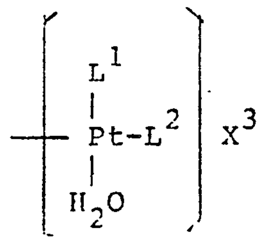

wherein L' and L² are as defined above and $X^3$ is nitrato or hydroxo or sulfato (said sulfato being the remainder sulfato resulting from binding of one of $X^1$ and $X^2$ to the activated dextran), and/or two hydrogen atoms of two $-\overset{\overset{O}{\|}}{C}-OH$ groups bound to one and the same carbon atom or to two neighboring carbon atoms as occurring at said active sites are, each independently, partly substituted by a group of the general formula

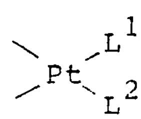

wherein L' and L² are as defined above, and/or a salt thereof, and, as required, reacting the thus-obtained dextran derivative and/or a salt thereof with an anion-forming compound to obtain a dextran derivative having one or more carboxylated active sites resulting from reaction with an aliphatic di- to tetrabasic acid or a reactive derivative thereof, wherein one hydrogen atom of the group of the formula

$-\overset{\overset{O}{\|}}{C}-OH$

occurring at said active sites is partly substituted by a group of the general formula

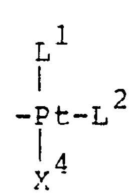 or 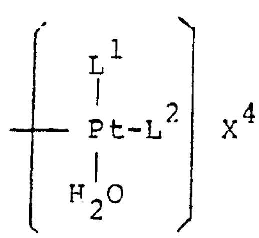

wherein L' and L² are as defined above and $X^4$ is an anionic ligand, and/or two hydrogen atoms of two $-\overset{\overset{O}{\|}}{C}-OH$ groups bound to one and the same carbon atom or to two neighboring carbon atoms as occurring at said active sites are, each independently, partly substituted by a group of the general formula

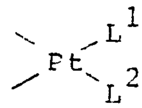

wherein L' and L² are as defined above, and/or a salt thereof.

4. A process for producing a hydroxyethyl starch derivative having one or more carboxylated active sites resulting from introduction of reaction with an aliphatic di- to tetrabasic acid or a reactive derivative thereof, wherein one hydrogen atom of the group of the formula

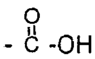

occurring at said active sites is partly substituted by a group of the general formula

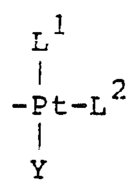 or 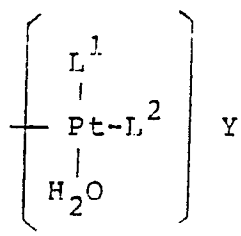

wherein L' and L² each independently is ammine or a unidentate ligand amine or combinedly represent a bidentate ligand amine and Y is an anionic ligand, and/or two hydrogen atoms of two 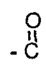 groups bound to one and the same carbon atom or to two neighboring carbon atoms as occurring at said active sites are, each independently, partly substituted by a group of the general formula

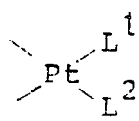

wherein L' and L² are as defined above, and/or a pharmacologically acceptable salt thereof which comprises reacting hydroxyethyl starch with an aliphatic di-, tri- or tetrabasic acid or a reactive derivative thereof, then reacting the thus-obtained activated hydroxyethyl starch; with a cis-platinum(II) complex of the general formula

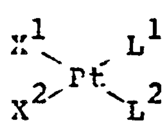

wherein L' and L² are as defined above and X' and X² each is nitrato or hydroxo or combinedly represent sulfato, to obtain a hydroxyethyl starch derivative having one or more carboxylated active sites resulting from reaction with an aliphatic di- to tetrabasic acid or a reactive derivative thereof, wherein one hydrogen atom of the group of the formula

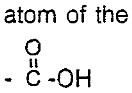

occurring at said active sites is partly substituted by a group of the general formula

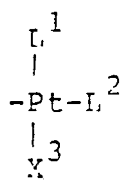 or 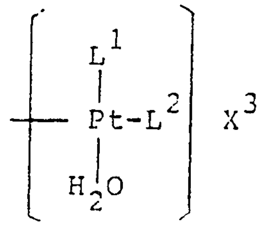

wherein $L^1$ and $L^2$ are as defined above and $X^3$ is nitrato or hydroxo or sulfato (said sulfato being the remainder sulfato resulting from binding of one of $X^1$ and $X^2$ to the activated hydroxyethyl starch), and/or two hydrogen atoms of two $-\overset{O}{\overset{\|}{C}}-OH$ groups bound to one and the same carbon atom or to two neighboring carbon atoms as occurring at said active sites are, each independently, partly substituted by a group of the general formula

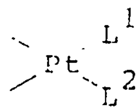

wherein $L^1$ and $L^2$ are as defined above, and/or a salt thereof, and, as required, reacting the thus-obtained hydroxyethyl starch derivative and/or a salt thereof with an anion-forming compound to obtain a hydroxyethyl starch derivative having one or more carboxylated active sites resulting from reaction with an aliphatic di - to tetrabasic acid or a reactive derivative thereof, wherein one hydrogen atom of the group of the formula

$-\overset{O}{\overset{\|}{C}}-OH$

occurring at said active sites is partly substituted by a group of the general formula

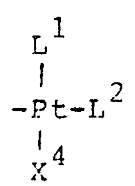 or 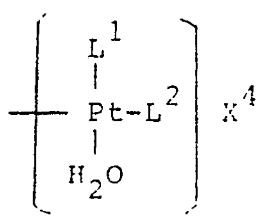

wherein $L^1$ and $L^2$ are as defined above and $X^4$ is an anionic ligand, and/or two hydrogen atoms of two $-\overset{O}{\overset{\|}{C}}-OH$ groups bound to one and the same carbon atom or to two neighboring carbon atoms as occurring at said active sites are, each independently, partly substituted by a group of the general formula

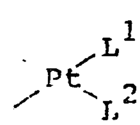

wherein $L^1$ and $L^2$ are as defined above, and/or a salt thereof.

5. A medicament which comprises a dextran or hydroxyethyl starch derivative or a pharmacologically acceptable salt thereof an claimed in Claim 1 or Claim 2.

6. An anticancer agent which comprises a dextran or hydroxyethyl starch derivative or a pharmacologically acceptable salt thereof as claimed in Claim 1 or Claim 2.

7. A use of a dextran or hydroxyethyl starch derivative or a pharmacologically acceptable salt thereof as claimed in Claim 1 or Claim 2 for manufacturing an anticancer agent.

Figure 1

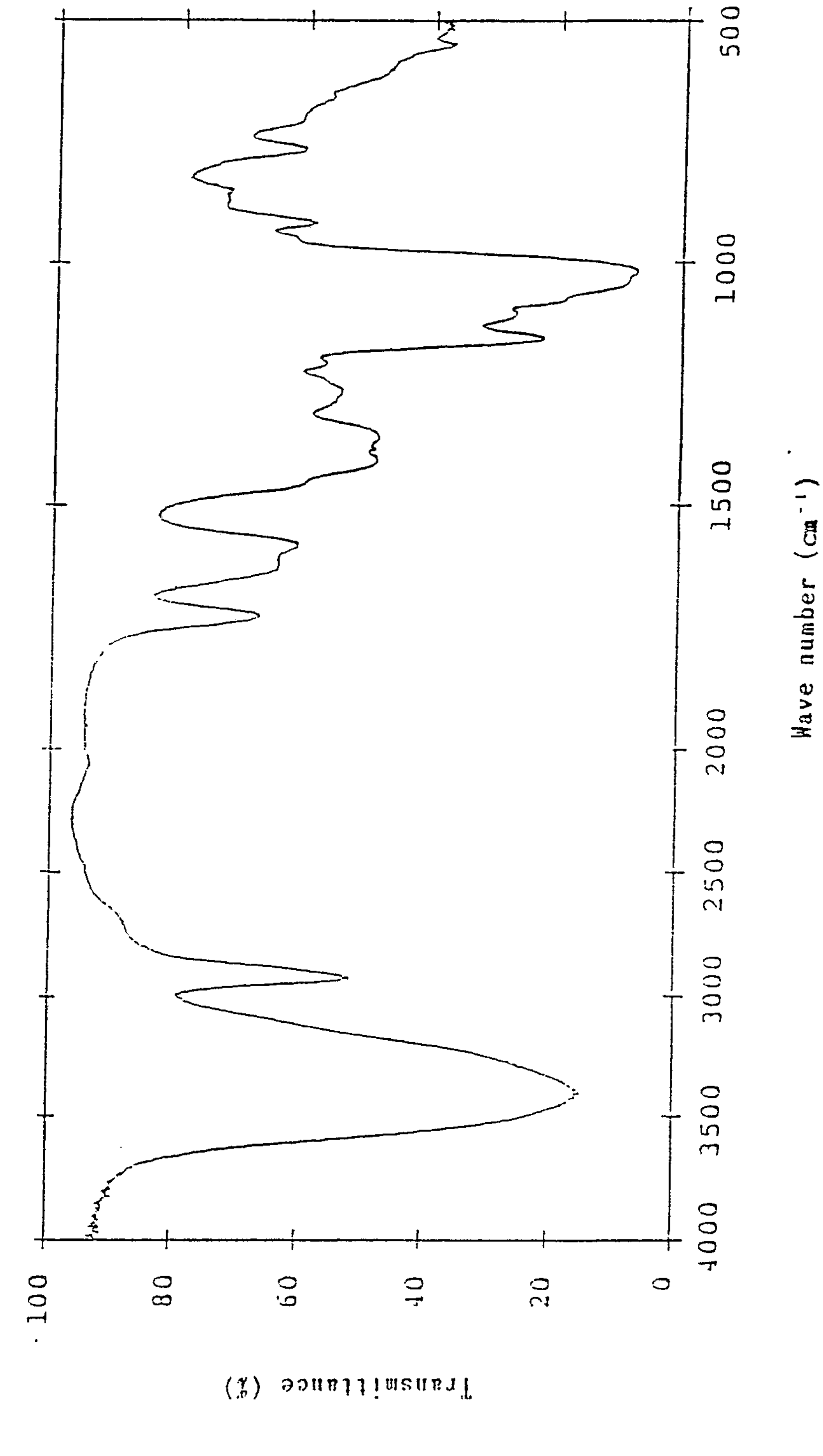

Transmittance (%)
100
80
60
40
20
0
4000
3500
3000
2500
2000
1500
1000
500
Wave number (cm⁻¹)

Figure 2

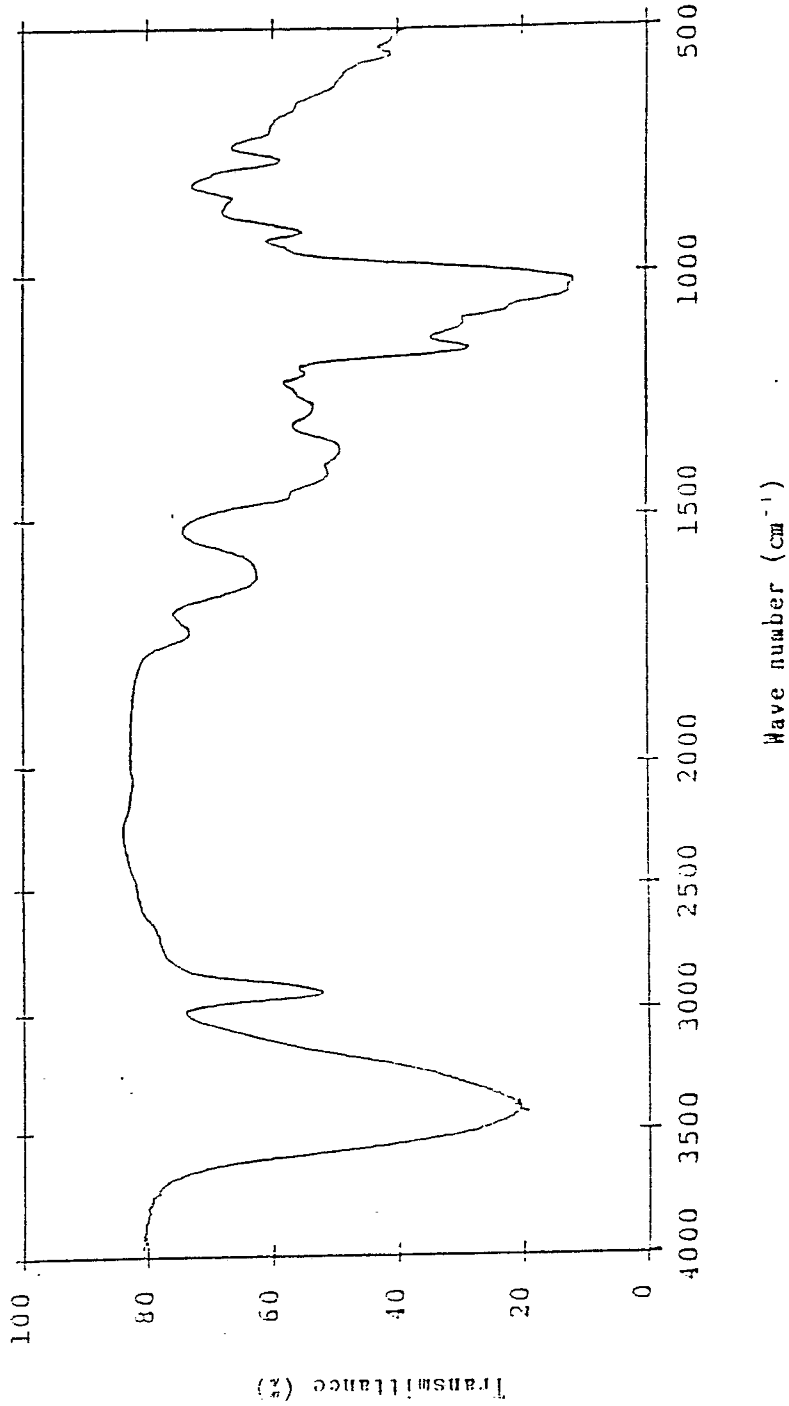
100
80
60
40
20
0
Transmittance (%)
4000
3500
3000
2500
2000
1500
1000
500
Wave number (cm-1)

Figure 3

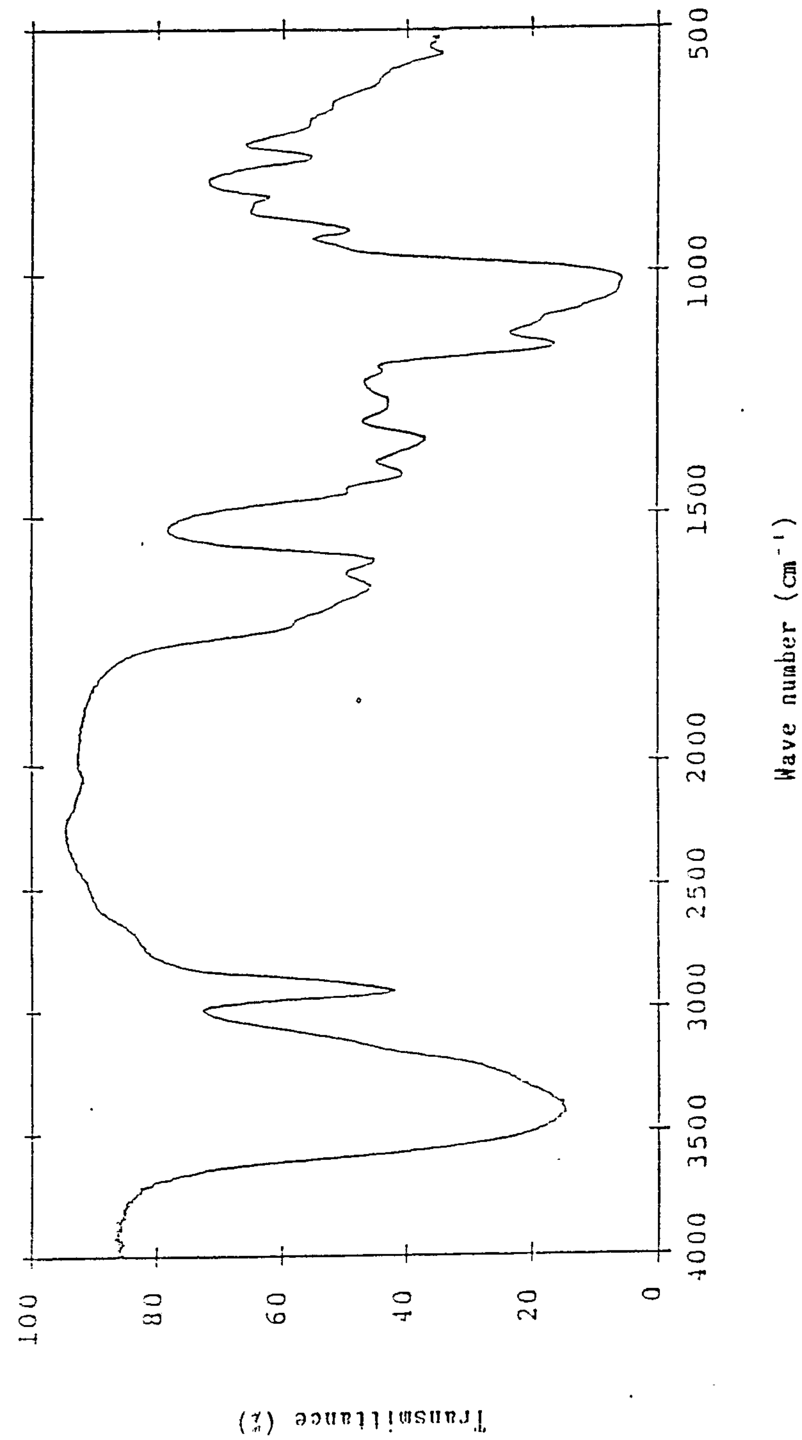
Transmittance (%)
100
80
60
40
20
0
Wave number (cm⁻¹)
4000
3500
3000
2500
2000
1500
1000
500

Figure 4

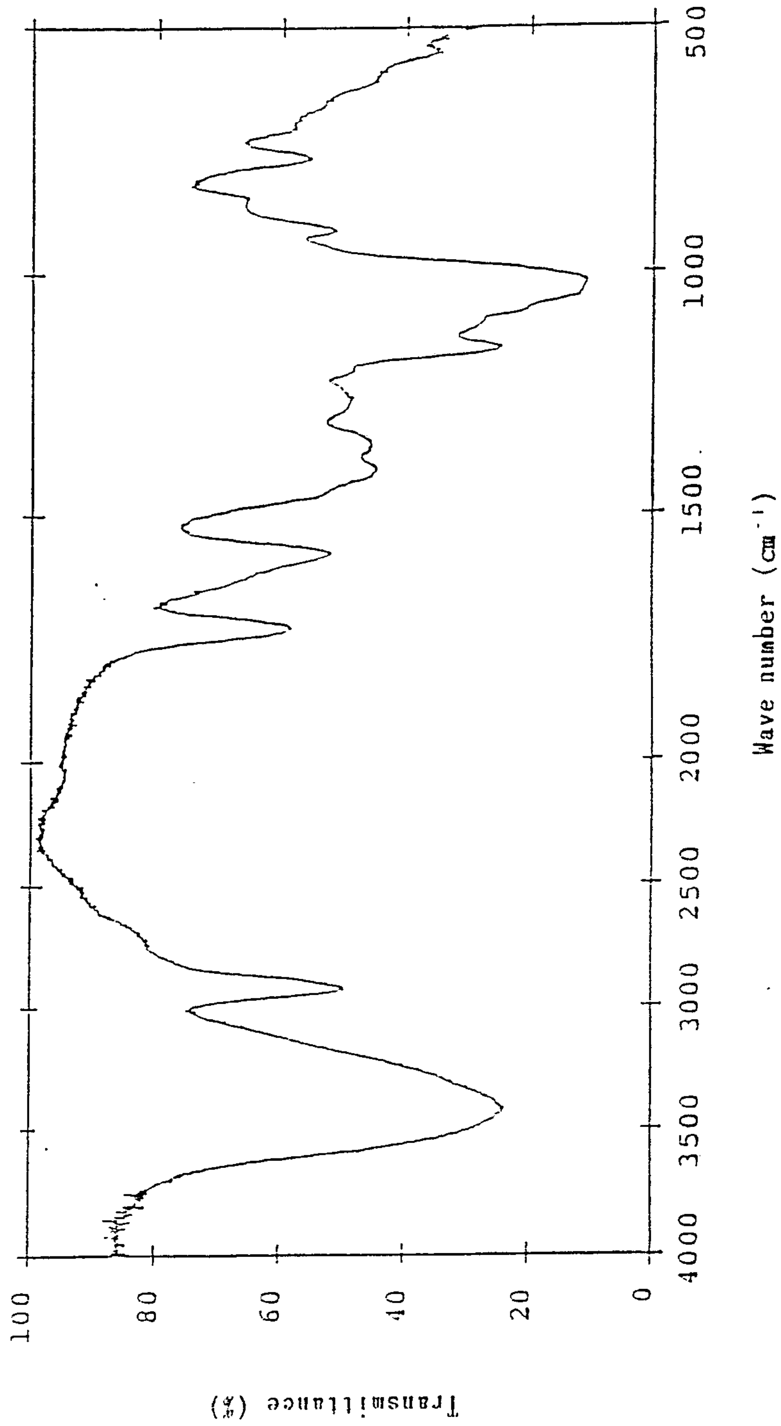
Transmittance (%)
100
80
60
40
20
0
4000
3500
3000
2500
2000
1500
1000
500
Wave number (cm⁻¹)

Figure 5

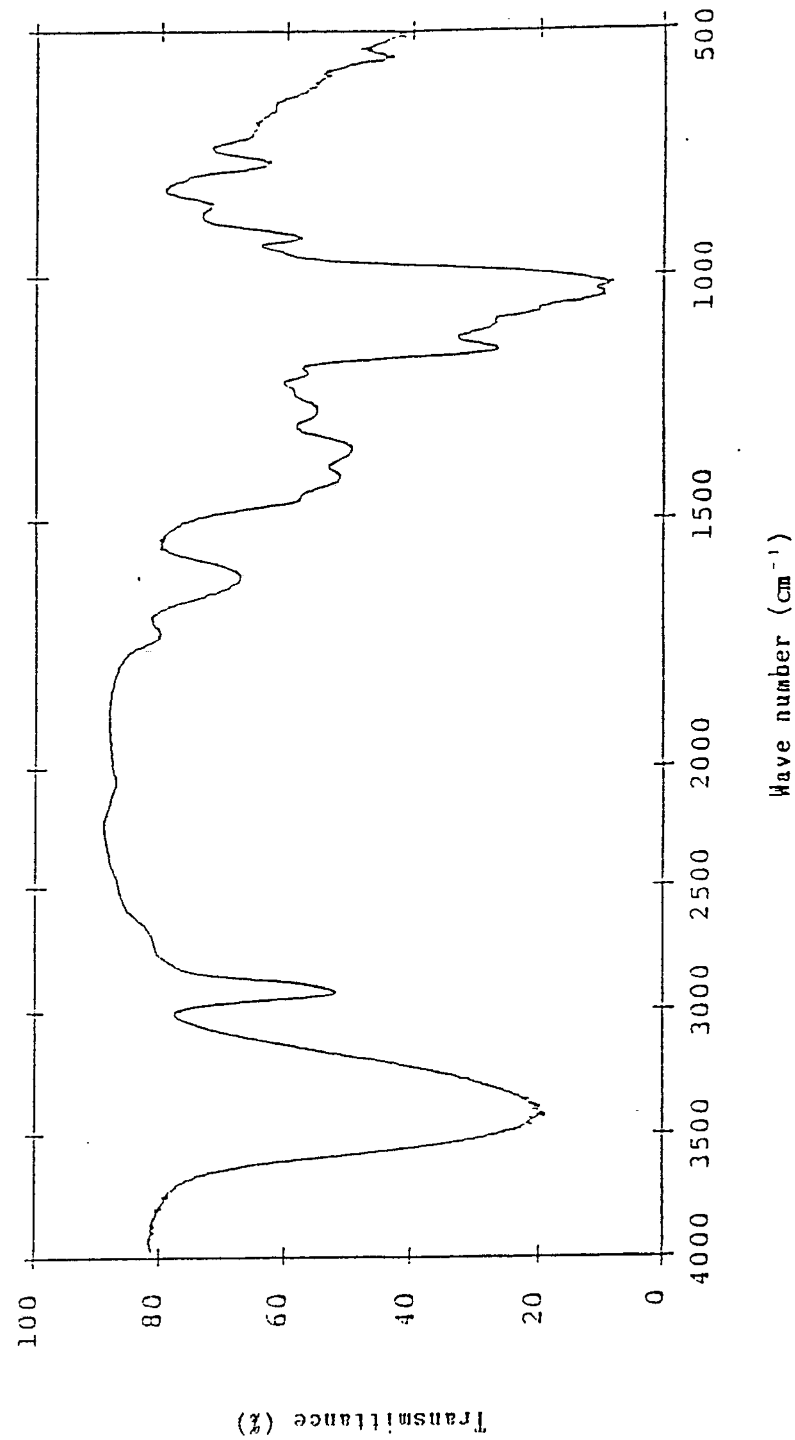
Transmittance (%)
100
80
60
40
20
0
Wave number (cm⁻¹)
4000
3500
3000
2500
2000
1500
1000
500

Figure 6

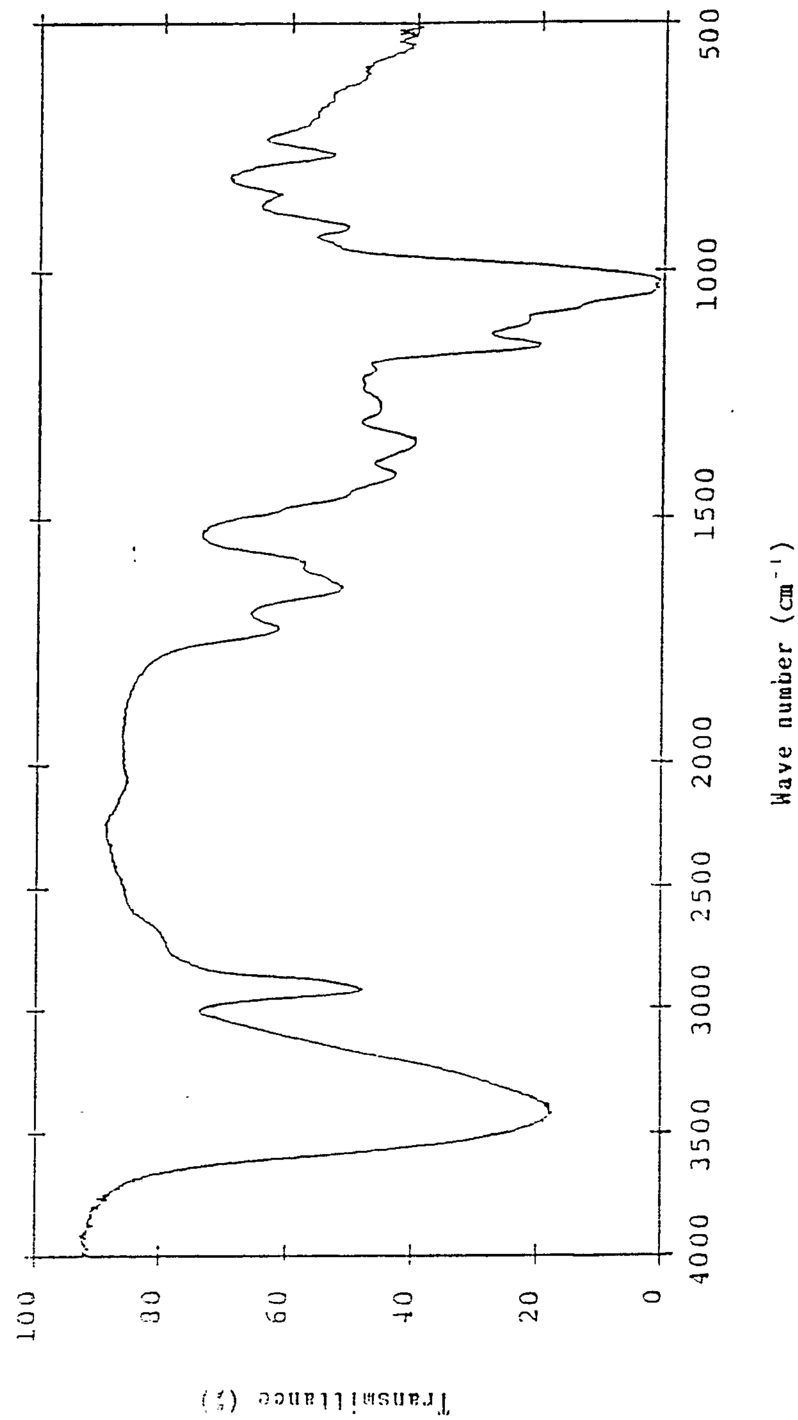
Transmittance (%)
100
80
60
40
20
0
Wave number (cm⁻¹)
4000
3500
3000
2500
2000
1500
1000
500

Figure 7

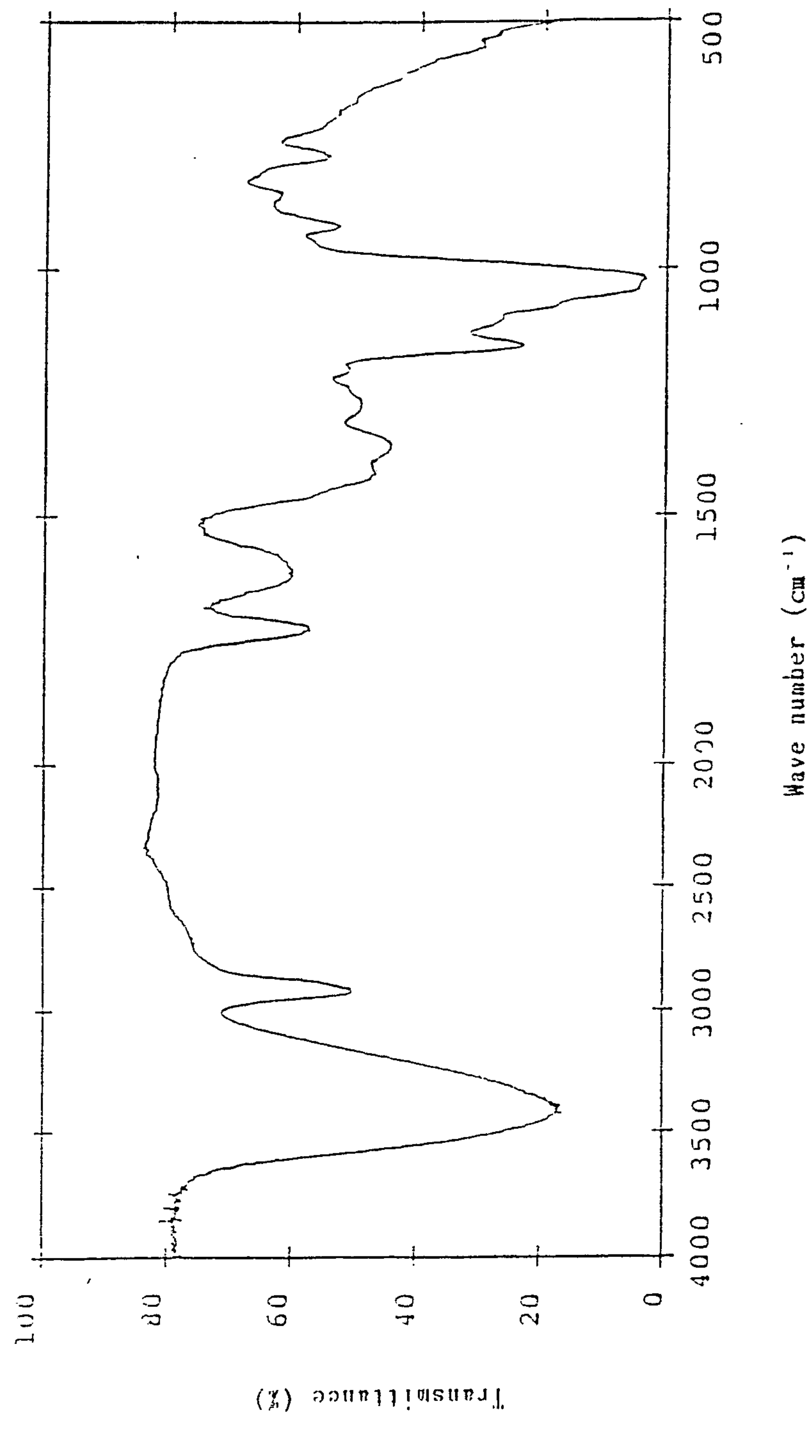
Transmittance (%)
100
80
60
40
20
0
4000
3500
3000
2500
2000
1500
1000
500
Wave number (cm⁻¹)

Figure 8

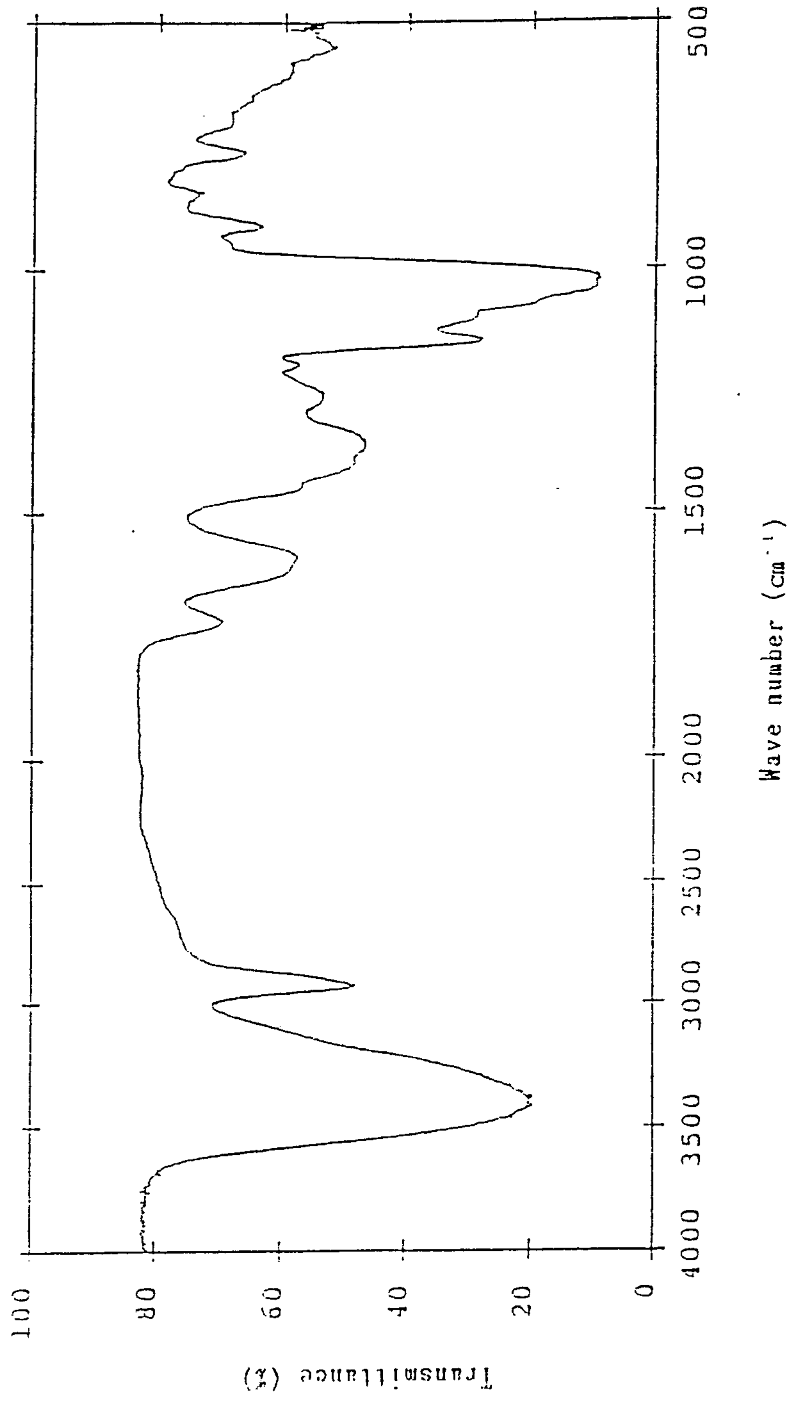

Transmittance (%)
100
80
60
40
20
0
4000
3500
3000
2500
2000
1500
1000
500
Wave number ($cm^{-1}$)

Figure 9

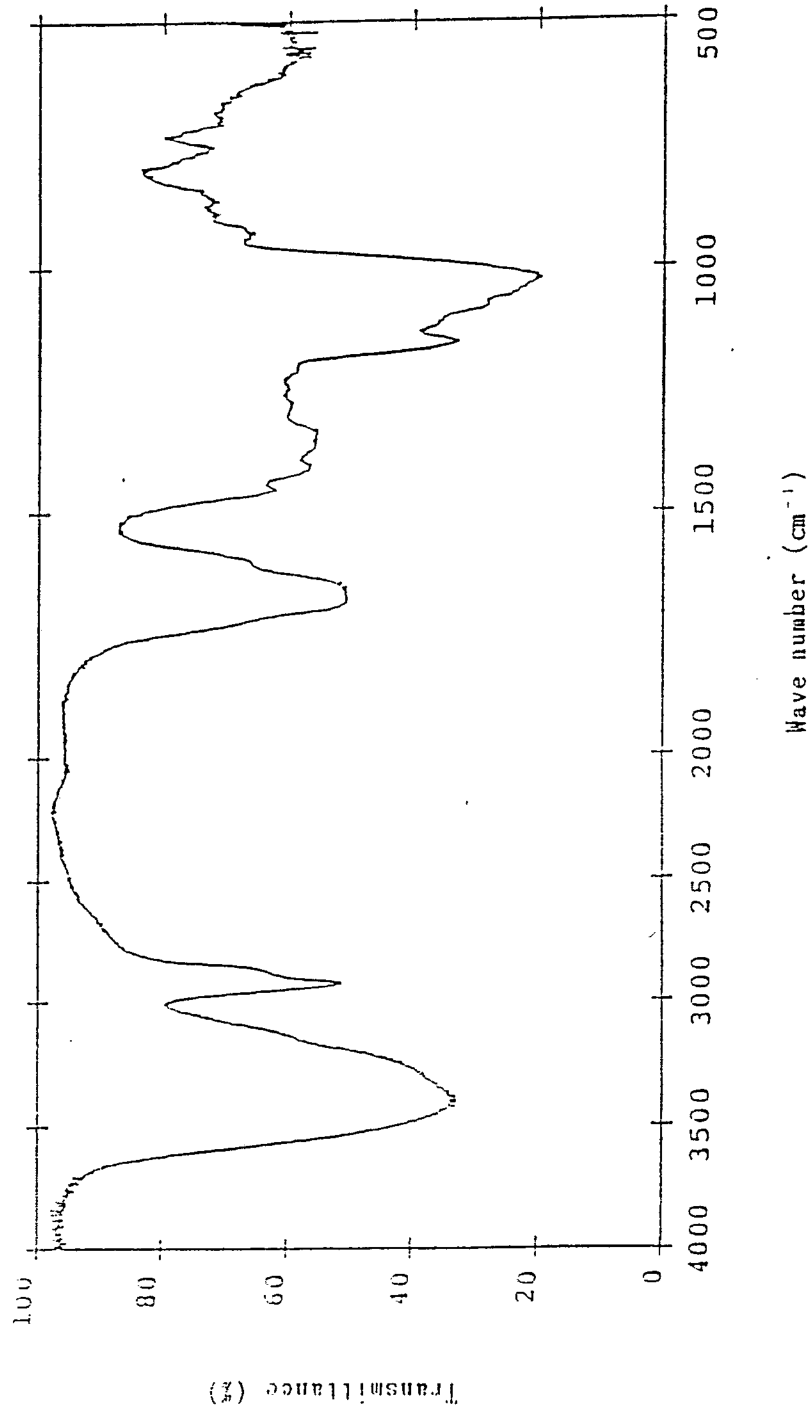

500
1000
1500
2000
2500
3000
3500
4000
Wave number (cm$^{-1}$)
100
80
60
40
20
0
Transmittance (%)

Figure 10

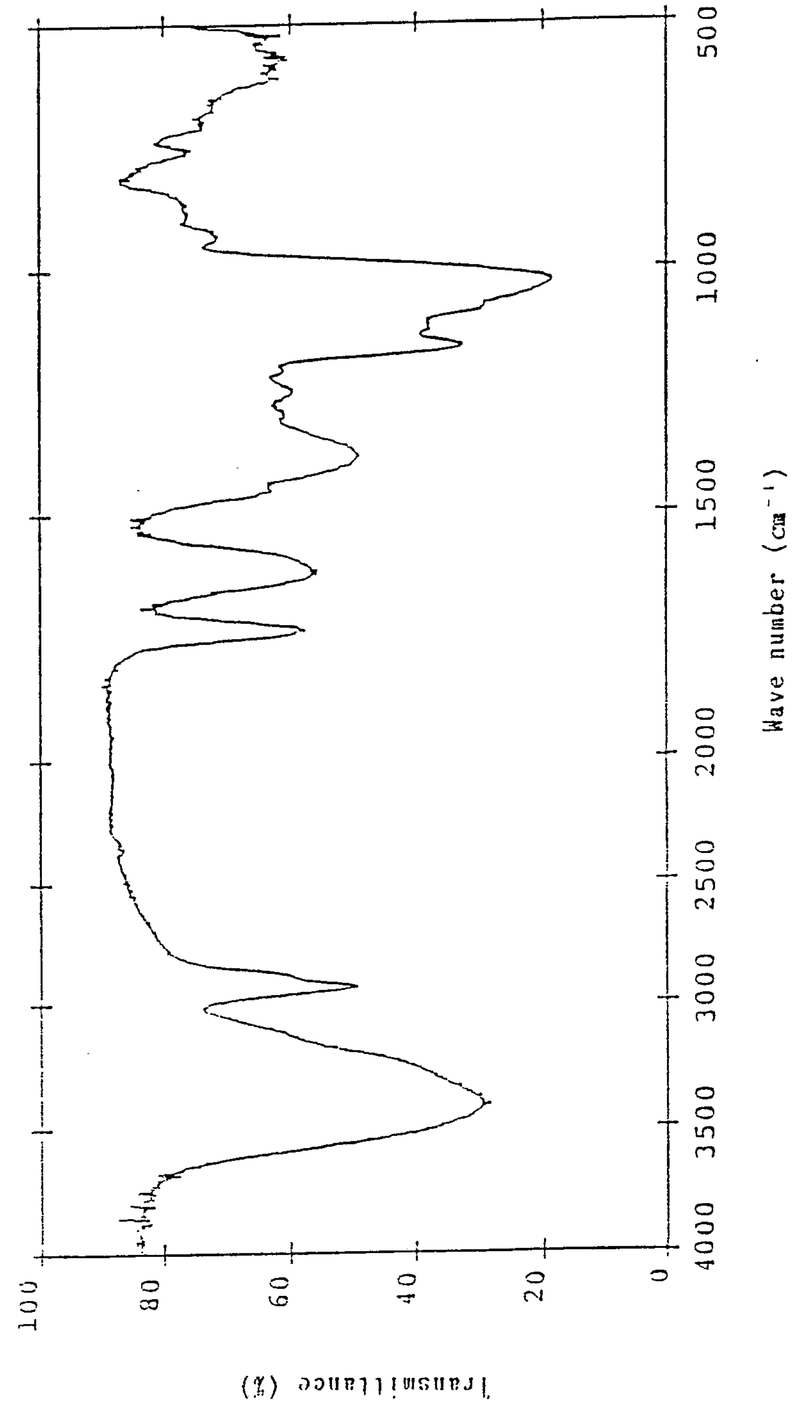

Transmittance (%)
100
80
60
40
20
0
Wave number (cm⁻¹)
4000
3500
3000
2500
2000
1500
1000
500

Figure 11

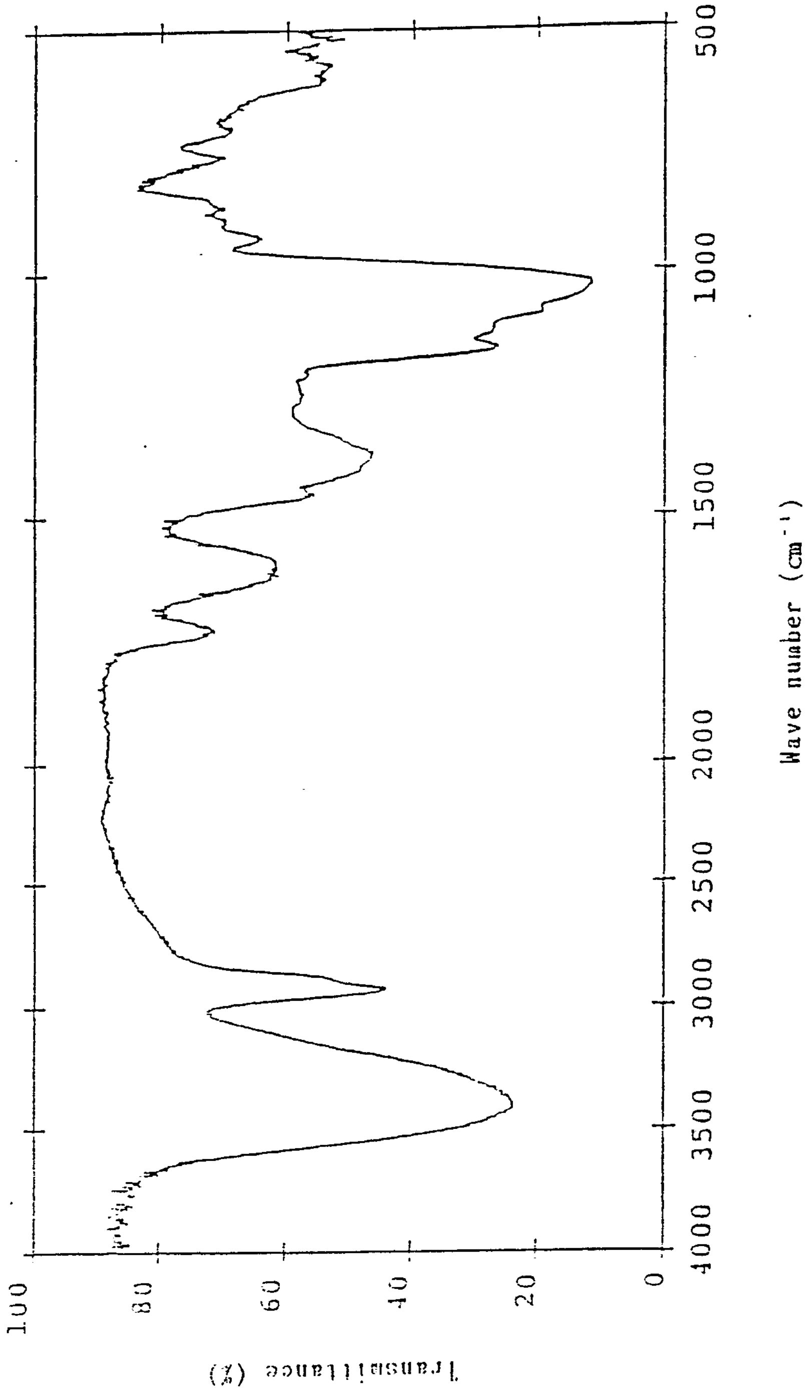
100
80
60
40
20
0
Transmittance (%)
4000
3500
3000
2500
2000
1500
1000
500
Wave number (cm⁻¹)

Figure 12

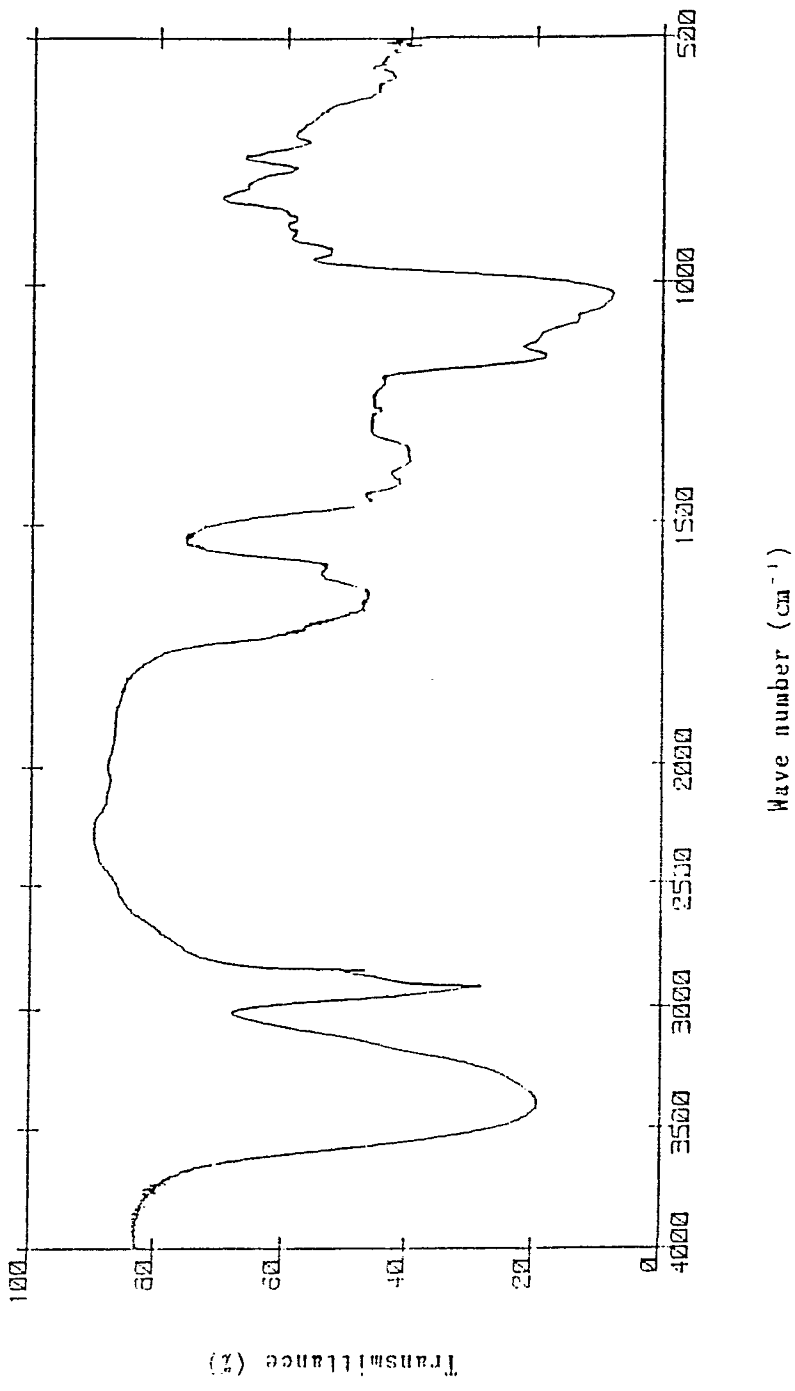
Transmittance (%)
100
80
60
40
20
0
4000
3500
3000
2500
2000
1500
1000
520
Wave number (cm⁻¹)

Figure 13

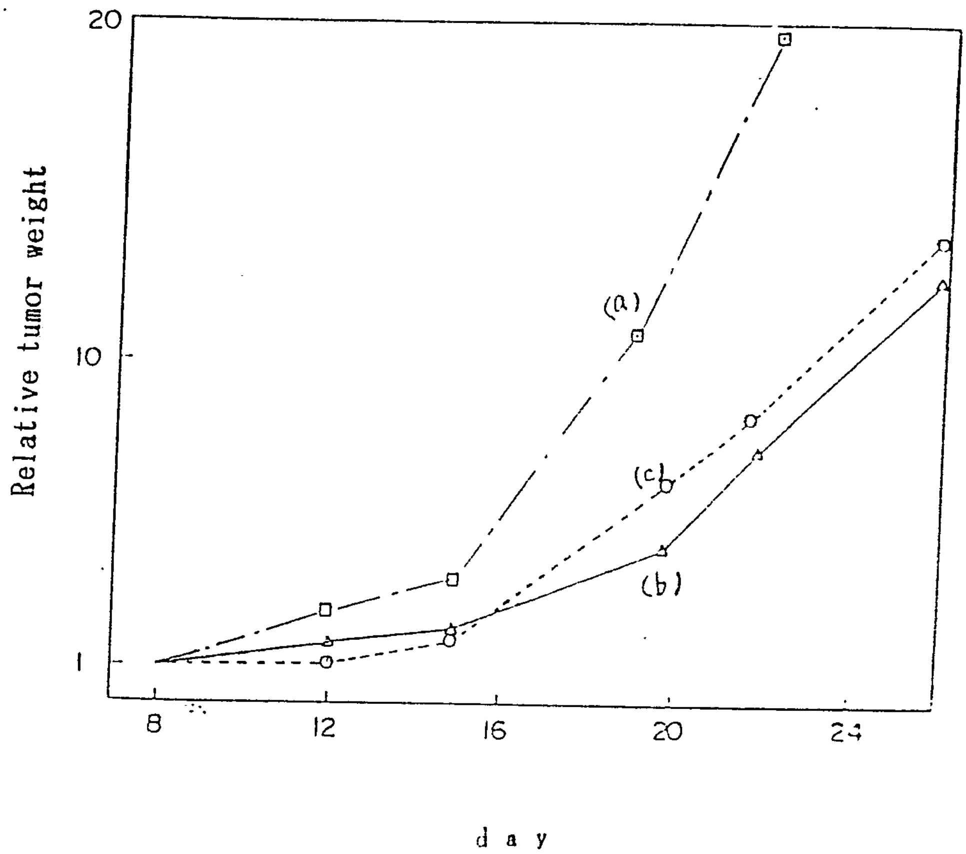

# Figure 14